(19) Europäisches Patentamt
European Patent Office
Office européen des brevets

(11) Veröffentlichungsnummer: **0 275 821 B1**

(12) **EUROPÄISCHE PATENTSCHRIFT**

(45) Veröffentlichungstag der Patentschrift: **26.02.92**

(51) Int. Cl.$^5$: **C07F 9/547**, A61K 31/675

(21) Anmeldenummer: **87810664.0**

(22) Anmeldetag: **16.11.87**

Die Akte enthält technische Angaben, die nach dem Eingang der Anmeldung eingereicht wurden und die nicht in dieser Patentschrift enthalten sind.

(54) **Neue substituierte Alkandiphosphonsäuren.**

(30) Priorität: **21.11.86 CH 4666/86**

(43) Veröffentlichungstag der Anmeldung:
**27.07.88 Patentblatt 88/30**

(45) Bekanntmachung des Hinweises auf die Patenterteilung:
**26.02.92 Patentblatt 92/09**

(84) Benannte Vertragsstaaten:
**AT BE CH DE ES FR GB GR IT LI LU NL SE**

(56) Entgegenhaltungen:
**EP-A- 170 228**
**EP-A- 0 084 822**
**EP-A- 0 258 618**
**DE-A- 3 203 307**

(73) Patentinhaber: **CIBA-GEIGY AG**
**Klybeckstrasse 141**
**CH-4002 Basel(CH)**

(72) Erfinder: **Jaeggi, Knut A., Dr.**
**General Guisan-Strasse 44**
**CH-4054 Basel(CH)**
Erfinder: **Widler, Leo, Dr.**
**Melchior-Berristrasse 11**
**CH-4142 Münchenstein(CH)**

**EP 0 275 821 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Alkandiphosphonsäuren, insbesondere Heteroarylalkandiphosphonsäuren der Formel

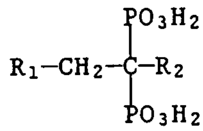

worin $R_1$ einen Imidazolyl-, 2H-1,2,3-, 1H-1,2,4- oder 4H-1,2,4-Triazolyl-, Tetrazolyl-, Oxazolyl-, Isoxazolyl-, Oxadiazolyl-, Thiazolyl- oder Thiadiazolylrest bedeutet, der unsubstituiert durch Niederalkyl, durch Niederalkoxy, durch Phenyl, welches seinerseits durch Niederalkyl, Niederalkoxy und/oder Halogen mono- oder disubstituiert sein kann, durch Hydroxy, durch Diniederalkylamino, durch Niederalkylthio und/oder durch Halogen C-mono- oder disubstituiert und an einem substituierbaren N-Atom durch Niederalkyl oder durch Phenylniederalkyl, welches seinerseits im Phenylteil durch Niederalkyl, Niederalkoxy und/oder Halogen mono- oder disubstituiert sein kann, N-substituiert ist, alk Niederalkylen bedeutet und $R_2$ Wasserstoff, Hydroxy, Amino, Niederalkylthio oder Halogen bedeutet, wobei nieder Reste bis und mit 7 C-Atome aufweisen, mit den Massgaben, dass $R_1$ von Imidazol-4-yl, 2-Methylthiazol-4-yl, 2-Methylthiazol-5-yl und 1,2,5-Thiadiazol-4-yl verschieden ist, wenn $R_2$ Wasserstoff darstellt, dass $R_1$ von Imidazol-2-yl, 5(4)-Methylimidazol-4(5)-yl, 2H-1,2,3-Triazol-4-yl, 1H-1,2,4-Triazol-1-yl, 1H-1,2,4-Triazol-3-yl, 5-Aethoxy-, 5-Aethoxy-2-methyl-, 2-Chlor-, 5-Methoxy- und 2,5-Dimethyloxazol-4-yl, 3-Methyl- und 3-Phenylisoxazol-5-yl, 3-Methyl-1,2,5-oxadiazol-4-yl,2-Methyl-1,3,4-oxadiazol-5-yl, 3-Phenyl-1,2,4-oxadiazol-5-yl, 2-Chlor- und 2-Methylthiazol-5-yl, 1,2,3- und 1,2,5-Thiadiazol-4-yl, 1,2,3-Thiadiazol-5-yl, 4-Methyl-1,2,3-thiadiazol-5-yl und 3-Methyl- und 3-Phenyl-1,2,4-thiadiazol-5-yl verschieden ist, wenn $R_2$ Hydroxy darstellt, und dass $R_1$ von Imidazol-4-yl verschieden ist, wenn $R_2$ Amino oder Dimethylamino darstellt, und ihre Salze, Verfahren zur Herstellung der erfindungsgemässen Verbindung, diese enthaltende pharmazeutische Präparate und ihre Verwendung als Arzneimittelwirkstoffe.

Imidazolyl ist z.B. Imidazol-1-yl, -2-yl oder -4-yl; Thiazolyl ist z.B. Thiazol-2-yl oder -4-yl; Oxazolyl ist z.B. Oxazol-2-yl oder -4-yl; Isoxazolyl ist z.B. Isoxazol-3-yl oder -4-yl; Triazolyl ist z.B. 1H-1,2,4-Triazol-1-yl, 4H-1,2,4-Triazol-3-yl oder -4-yl oder 2H-1,2,3-Triazol-4-yl; Tetrazolyl ist z.B. Tetrazol-5-yl; Thiadiazolyl ist z.B. 1,2,5-Thiadiazol-3-yl und Oxadiazolyl ist z.B. 1,3,4-Oxadiazol-2-yl. Die genannten Reste können einen oder zwei, gleiche oder verschiedene, der eingangs genannten Substituenten aufweisen. Gegebenenfalls wie angegeben substituierte Reste $R_1$ sind beispielsweise unsubstituierte oder durch im Phenylteil unsubstituiertes oder wie angegeben substituiertes Phenyl C-substituierte bzw. durch $C_1$-$C_4$-Alkyl, wie Methyl, C- oder N-substituierte Imidazol-2- oder -4-ylreste, z.B. Imidazol-2-yl, 1-$C_1$-$C_4$-Alkyl-, wie 1-Methylimidazol-2-yl oder 2-oder 5-$C_1$-$C_4$-Alkyl-, wie 2-oder 5-Methylimidazol-4-yl, unsubstituierte Thiazolylreste, z.B. Thiazol-2-yl, oder unsubstituierte oder durch $C_1$-$C_4$-Alkyl, wie Methyl, substituierte 1H-1,2,4-Triazolylreste, z.B. 1-$C_1$-$C_4$-Alkyl-, wie 1-Methyl-1H-1,2,4-triazol-5-yl, oder unsubstituierte oder durch im Phenylteil unsubstituiertes oder wie angegeben substituiertes Phenyl bzw. durch $C_1$-$C_4$-Alkyl, wie Methyl, C-substituierte Imidazol-1-yl-, Pyrazol-1-yl-, 1H-1,2,4-Triazol-1-yl-, 4H-1,2,4-Triazol-4-yl- oder Tetrazol-1-ylreste, z.B. Imidazol-1-yl, 2-, 4- oder 5-$C_1$-$C_4$-Alkyl-, wie 2-, 4- oder 5-Methylimidazol-1-yl, Pyrazol-1-yl, 3- oder 4-$C_1$-$C_4$-Alkyl-, wie 3- oder 4-Methylpyrazol-1-yl, 1H-1,2,4-Tetrazol-1-yl, 3-$C_1$-$C_4$-Alkyl-, wie 3-Methyl-1H-1,2,4-triazol-1-yl, 4H-1,2,4-Triazol-4-yl, 3-$C_1$-$C_4$-Alkyl-, wie 3-Methyl-4H-1,2,4-triazol-4-yl oder 1H-Tetrazol-1-yl.

Nachstehend sind unter niederen Resten und Verbindungen beispielsweise solche zu verstehen, die bis und mit 7, insbesondere bis und mit 4, C-Atome aufweisen. Ferner haben die Allgemeinbegriffe beispielsweise folgende Bedeutungen:

Niederalkyl ist beispielsweise $C_1$-$C_4$-Alkyl, wie Methyl, Aethyl, Propyl oder Butyl, ferner Iso-, Sekundär- oder Tertiärbutyl, kann aber auch eine $C_5$-$C_7$-Alkyl-, wie Pentyl-, Hexyl- oder Heptylgruppe sein.

Phenylniederalkyl ist beispielsweise Phenyl-, vor allem 1-Phenyl-$C_1$-$C_4$-alkyl, wie Benzyl.

Niederalkoxy ist beispielsweise $C_1$-$C_4$-Alkoxy, wie Methoxy, Aethoxy, Propyloxy, Isopropyloxy, Butyloxy, Isobutyloxy, Sekundärbutyloxy oder Tertiärbutyloxy.

Diniederalkylamino ist beispielsweise Di-$C_1$-$C_4$-Alkylamino, wie Dimethylamino, Diäthylamino, N-Aethyl-N-methyl-amino, Dipropylamino, N-Methyl-N-propylamino oder Dibutylamino.

Niederalkylthio ist beispielsweise $C_1$-$C_4$-Alkylthio, wie Methylthio, Aethylthio, Propylthio oder Butylthio, ferner Iso-, Sekundär- oder Tertiärbutylthio.

Halogen ist beispielsweise Halogen der Atomnummer bis und mit 35, wie Fluor, Chlor oder Brom.

Salze von Verbindungen der Formel I sind insbesondere deren Salze mit pharmazeutisch verwendbaren Basen, wie nicht-toxische, von Metallen oder Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di-oder Triniederalkylaminen, z.B. Methyl-, Aethyl-, Dimethyl- oder Diäthylamin, Mono-, Di- oder Tri-(hydroxyniederalyl)-aminen, wie Aethanol-, Diäthanol- oder Triäthanolamin, Tris-(hydroxymethyl)-amino-methanoder 2-Hydroxytertiärbutylamin, oder N-(Hydroxyniederalkyl)-N,N-diniederalkylaminen bzw. N-(Polyhydroxyniederalkyl)-N-niederalkylaminen, wie 2-(Dimethylamino)-äthanol oder D-Glucamin, oder quaternären aliphatischen Ammoniumhydroxiden, z.B. mit Tetrabutylammoniumhydroxid.

In diesem Zusammenhang ist auch zu erwähnen, dass die Verbindungen der Formel I in Form innerer Salze vorliegen können, sofern die Gruppe $R_1$ genügend basisch ist. Diese Verbindungen können dementsprechend auch durch Behandlung mit einer starken Protonensäure, wie mit einer Halogenwasserstoffsäure, Schwefelsäure, Sulfonsäure, z.B. Methan-oder p-Toluolsulfonsäure, oder Sulfaminsäure, z.B. N-Cyclohexylsulfaminsäure, in die entsprechenden Säureadditionssalze überführt werden.

Die Verbindungen der Formel I und ihre Salze weisen wertvolle pharmakologische Eigenschaften auf. Insbesondere besitzen sie eine ausgeprägte regulierende Wirkung auf den Calcium-Stoffwechsel von Warmblütern. Insbesondere bewirken sie an der Ratte eine ausgeprägte Hemmung der Knochenresorption, die sich sowohl in der Versuchsanordnung gemäss Acta Endocrinol. 78, 613-24 (1975) anhand des PTH-induzierten Anstieges des Serumcalciumspiegels nach subcutaner Applikation in Dosen von etwa 0,01 bis etwa 1,0 mg/kg, als auch im TPTX (Thyroparathyroidectomised) - Rattenmodell anhand der durch Vitamin $D_3$ ausgelösten experimentellen Hypercalcämie nach Gabe von Dosen von etwa 0,0003 bis 1.0 mg s.c. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp. Med. 159, 1388-96 (1984) in Dosen von etwa 0,001 bis 1,0 mg/kg s.c. eine deutliche Hemmung auf das Fortschreiten chronisch-arthritischerProzesse. Sie sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, der Neuritis, der Bursitis, Periodontitis und der Tendinitis, der Fibrodysplasie, der Osteoarthrose oder der Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, morbus Paget und die Osteodystrophia fibrosa, ebenso durch Tumore ausgelöste osteolytische Prozesse.

Aus der DE-A-3 203 307 und der EP-A-84 822 waren bereits in 1,4-Stellung des Pyrazolteiles durch gegebenenfalls substitutierte Phenylreste, substituierte 1-hydroxy-2-(pyarazo-3-yl)-äthan-1,1-diphosphonsäuren und in EP-A-170 228 in ω-Stellung durch einen Imidazol-, Oxazol- oder Isoxazolrest in 1-Stellung gegebenenfalls durch Hydroxy, Amino oder Niederalkylamino substituierte $C_3$-$C_9$-Alkan-1,1-diphosphonsäuren mit der Calciumstoffwechsel regulierender Wirkung vorbekannt. Ferner sind in der nicht vorpublizierten EP-A-258 618 in ω-Stellung durch einen heteroaromatischen Rest und in 1-Stellung gegebenenfalls durch Hydroxy, Amino oder Niederalkylamino substituierte Alkandiphosphonsäuren mit ähnlichen Eigenschaften beschrieben.

Die Erfindung betrifft in erster Linie Verbindungen der Formel I, worin $R_1$ einen unsubstituierten oder durch Niederalkyl, Niederalkoxy, unsubstituiertes oder durch Niederalkyl, Niederalkoxy und/oder Halogen mono- oder disubstituiertes Phenyl, Hydroxy, Diniederalkylamino, Niederalkylthio und/oder Halogen C-mono- oder disubstituierten und/oder an einem substituierbaren N-Atom durch Niederalkyl oder unsubstituiertes oder durch Niederalkyl, Niederalkoxy und/oder Halogen mono- oder disubstituiertes Phenylniederalkyl N-substituierten Imidazolyl-, 2H-1,2,3- oder 4H-1,2,4-Triazolyl-, Tetrazolyl-, Oxazolyl-, Isoxazolyl-, Oxadiazolyl-, Thiazolyl- oder Thiadiazolylrest bedeutet und $R_2$ Wasserstoff, Hydroxy, Amino, Niederalkylthio oder Halogen bedeutet, und ihre Salze, insbesondere ihre inneren Salze und pharmazeutisch verwendbaren Salze mit Basen.

Die Erfindung betrifft vorzugsweise einerseits Verbindungen der Formel I, worin $R_1$ einen unsubstituierten oder durch Phenyl C-substituierten bzw. durch $C_1$-$C_4$-Alkyl, wie Methyl, C- oder N-substituierten Imidazol-2- oder -4-ylrest, z.B. Imidazol-2-yl, 1-$C_1$-$C_4$-Alkyl-, wie 1-Methylimidazol-2-yl oder 2- oder 5-$C_1$-$C_4$-Alkyl-, wie 2- oder 5-Methylimidazol-4-yl, oder einen unsubstituierten oder durch $C_1$-$C_4$-Alkyl, wie

Methyl, substituierten 1H-1,2,4-Triazolylrest darstellt, und $R_2$ Hydroxy oder in zweiter Linie Wasserstoff bedeutet, und ihre Salze, insbesondere pharmazeutisch verwendbare Salze.

Die Erfindung betrifft vorzugsweise andererseits Verbindungen der Formel I, worin $R_1$ 2-, 4- oder 5-$C_1$-$C_4$-Alkyl-, wie 2-, 4- oder 5-Methylimidazol-1-yl, 3-$C_1$-$C_4$-Alkyl-, wie 3-Methyl-1H-1,2,4-triazol-1-yl, 4H-1,2,4-Triazol-4-yl, 3-$C_1$-$C_4$-Alkyl-, wie 3-Methyl-4H-1,2,4-triazol-4-yl oder 1H-Tetrazol-1-yl darstellt, und $R_2$ Hydroxy oder in zweiter Linie Wasserstoff bedeutet, und ihre Salze, insbesondere pharmazeutisch verwendbare Salze.

Die Erfindung betrifft weiterhin Verbindungen der Formel I, worin $R_1$ einen unsubstituierten oder durch $C_1$-$C_4$-Alkyl, $C_1$-$C_4$-Alkoxy, Phenyl, Hydroxy, Di-$C_1$-$C_4$-Alkylamino, $C_1$-$C_4$-Alkylthio und/oder Halogen der Atomnummer bis und mit 35 C-mono- oder di-substituierten und/oder durch $C_1$-$C_4$-Alkyl oder Phenyl-$C_1$-$C_4$-alkyl N-substituierten Imidazolyl-, 4H-1,2,4-Triazol-4-yl- oder Thiazolylrest bedeutet und $R_2$ Hydroxy, Wasserstoff oder Amino bedeutet, und ihre Salze.

Die Erfindung betrifft weiter auch die Verbindungen der Formel I, worin $R_1$ einen unsubstituierten oder durch Phenyl C-substituierten bzw. durch $C_1$-$C_4$-Alkyl, C- oder N-substituierten Imidazol-2- oder -4-ylrest, einen unsubstituierten Thiazolylrest oder einen unsubstituierten oder durch $C_1$-$C_4$-Alkyl N-substituierten 1H-1,2,4-Triazolylrest darstellt und $R_2$ Hydroxy oder Wasserstoff bedeutet, und ihre Salze und die Verbindungen gemäss Anspruch 1, worin $R_1$ Imidazol-1-yl, 2-, 4- oder 5-$C_1$-$C_4$-Alkylimidazol-1-yl, 3-$C_1$-$C_4$-Alkyl-1H-1,2,4-triazol-1-yl, 4H-Triazol-4-yl, 3-$C_1$-$C_4$-Alkyl-4H-1,2,4-triazol-4-yl oder 1H-Tetrazol-1-yl bedeutet und alk sowie $R_2$ die in Anspruch 1 angegebenen Bedeutungen haben, und ihre Salze.

Die Erfindung betrifft in allererster Linie Verbindungen der Formel I, worin $R_1$ einen unsubstituierten oder durch Phenyl bzw. durch $C_1$-$C_4$-Alkyl, wie Methyl, C-substituierten Imidazol-1-yl-, 4H-1,2,4-Triazol-4-yl- oder Tetrazo-1-ylrest darstellt und $R_2$ Hydroxy oder in zweiter Linie Wasserstoff bedeuten, und ihre Salze, insbesondere pharmazeutisch verwendbare Salze.

Die Erfindung betrifft namentlich die in den Beispielen genannten Verbindungen der Formel I und ihre Salze, insbesondere ihre inneren Salze und pharmazeutisch verwendbaren Salze mit Basen.

Die Erfindung betrifft weiterhin ein auf an sich bekannten Methoden beruhendes Verfahren zur Herstellung von Verbindungen der Formel I und ihrer Salze. Dieses ist dadurch gekennzeichnet, dass man

a) in einer an einem substituierbaren N-Atom des Restes $R_1$ gegebenenfalls intermediär geschützten Verbindung der Formel

$$R_1 - CH_2 - \underset{X_2}{\overset{X_1}{C}} - R_2 \qquad (II),$$

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt oder

b) eine an einem substituierbaren N-Atom des Restes $R_1$ gegebenenfalls intermediär geschützte Verbindung der Formel

$R_1$ - $CH_2$ - $X_3$ (III),

worin $X_3$ eine Carboxy-, Carbamyl-, Iminoäther-, Iminoester- oder Cyanogruppe bedeutet, mit phosphoriger Säure und Phosphortrichlorid umsetzt, wobei ausgehend von Verbindungen der Formel III, worin $X_3$ eine Carbamyl-, Iminoäther-, Iminoester- oder Cyanogruppe ist, bei der hydrolytischen Aufarbeitung Verbindungen der Formel I erhalten werden, worin $R_2$ Amino ist, und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

Gemäss der Verfahrensvariante a) in Phosphono zu überführende funktionell abgewandelte Phosphonogruppen liegen beispielsweise in einer Esterform, insbesondere einer Diesterform der Formel $-P(=O)(OR)_2$ (IV) vor, worin OR beispielsweise Niederalkoxy oder eine gegebenenfalls durch Niederalkyl, Niederalkoxy, Halogen, Trifluormethyl und/oder Hydroxy substituierte Phenyloxygruppe bedeutet.

Die Ueberführung einer funktionell abgewandelten in die freie Phosphonogruppe erfolgt in üblicher Weise durch Hydrolyse, beispielsweise in Gegenwart einer Mineralsäure, wie Bromwasserstoff-, Chlorwasserstoff- oder Schwefelsäure, oder durch Umsetzung mit einem Triniederalkyl-halogensilan, z.B. mit Trimethylchlorsilan in Gegenwart von Natriumjodid oder insbesondere Trimethyljodsilan oder oder Trimethylbromsilan, vorzugsweise unter Kühlen, z.B. im Temperaturbereich von etwa 0° bis etwa 25°C.

Die Ausgangsstoffe der Formel II, worin $R_2$ Hydroxy oder Amino ist, können beispielsweise hergestellt werden, indem man eine Verbindung der Formel

$R_1$ - $CH_2$ - COOH (IIa)

oder vorzugsweise das Nitril oder Säurechlorid derselben mit einem entsprechenden Phosphorigsäuretriester der Formel $P(OR)_3$ (IIb),worin R beispielsweise Niederalkyl bedeutet, in Gegenwart eines Triniederalkylamins, z.B. Triäthylamin, zu einem Zwischenprodukt, mutmasslich einer Verbindung der Formel

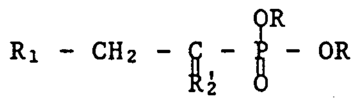

(IIc; $R_2'$ = Oxo, Imino)

umsetzt und diese mit einem Phosphorigsäurediester der Formel $H-P(=0)(OR)_2$ (IId) bzw. $P(OH)(OR)_2$ (IIe), worin R beispielsweise Niederalkyl bedeutet, in Gegenwart eines Diniederalkylamins, z.B. von Diäthylamin, oder eines Alkalimetallniederalkanolats, z.B. von Natriummethanolat, zur entsprechenden Verbindung der Formel

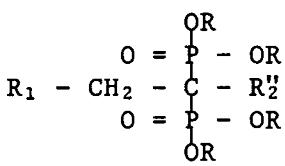

(IIf; $R_2''$ = Hydroxy, Amino)

weiterumsetzt. Verbindungen IIa werden beispielsweise erhalten indem man eine entsprechende Verbindung der Formel

$R_1$ - $CH_3$ (IIIa),

mit einer starken Base beispielsweise einer der unter der Verfahrensvariante a) genannten Metallbasen in das Carbeniatsalz überführt und dieses mit Kohlendioxid umsetzt oder indem man eine Verbindung der Formel

$R_1$-$CH_2$-Y (IIg),

worin Y reaktionsfähiges verestertes Hydroxy, insbesondere Halogen, wie Brom, bedeutet, mit einem Alkalimetallcyanid, z.B. mit Natriumoder Kaliumcyanid in das entsprechende Nitril (IIg; Y = CN) überführt und dieses zur Säure hydrolysiert, insbesondere unter basischen Bedingungen.

Ausgangsstoffe II, worin $R_2$ Wasserstoff ist, werden beispielsweise erhalten, indem man eine Verbindung der Formel

$R_1$-$CH_2$-Y (IIg),

worin Y reaktionsfähiges verestertes Hydroxy, insbesondere Halogen, wie Brom, bedeutet, in Gegenwart einer Metallbase, wie des Hydrides, eines Amides oder einer Kohlenwasserstoffverbindung eines Alkalimetalles, z.B. von Natriumhydrid, Natriumamid, Ditrimethylsilylnatriumamid oder Butyllithium, mit einem Methandiphosphonsäureester, z.B. der Formel

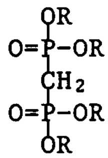

(IIh)

umsetzt, worin R beispielsweise Niederalkyl bedeutet.

Ausgangsstoffe der Formel II, worin der Rest $R_1$ über ein N-Atom gebunden ist und $R_2$ für Wasserstoff oder Hydroxy steht, können auch hergestellt werden, indem man eine entsprechende Verbindung der Formel

$R_1—H$ (IIi)

in Gegenwart einer starken Metallbase wie eines Alkalimetall- oder Erdalkalimetallhydrides, z.B. von Natriumhydrid, mit einer Verbindung der Formel

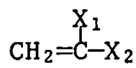 (IIj1) bzw. 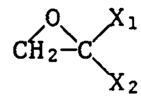 (IIj2),

worin $X_1$ und $X_2$ insbesondere Gruppen der Formel IV bedeuten, umsetzt.

Verbindungen II, worin $R_2$ Niederalkylthio oder Halogen bedeutet, können beispielsweise ausgehend von den entsprechenden Verbindungen II, worin $R_2$ Wasserstoff darstellt, hergestellt werden, indem man diese mit einer starken Base, z.B. einer der vorstehend genannten Metallbasen, in das Carbeniatsalz überführt und dieses mit einem Niederalkylthioüberträger, beispielsweise einem Diniederalkyldisulfid oder einem Niederalkansulfenylchlorid, bzw. einem Halogenüberträger, beispielsweise einem Halogen, z.B. Chlor oder Brom, Perchlorsäurefluorid ($FClO_3$) oder dergl., weiterumsetzt.

In Ausgangsstoffen der Formel III für die Verfahrensvariante b) sind Iminoäther- bzw. Iminoestergruppen beispielsweise solche der Formel -C(=NH)-$X_3'$ (III'), worin $X_3'$ veräthertes oder verestertes Hydroxy, wie Niederalkoxy, eine Phenoxygruppe, Niederalkanoyloxy, eine Benzoyloxygruppe oder ein Halogenatom, z.B. Chlor, bedeutet. Verbindungen III, worin $X_3$ eine der genannten Gruppen III' bedeutet, können auch als Salze, wie Mineralsäuresalze, z.B. als Hydrohalogenide vorliegen.

Die Umsetzung von Verbindungen der Formel III mit phosphoriger Säure und Phosphortrichlorid erfolgt in üblicher Weise, wobei die Phosphorigsäurekomponente vorzugsweise durch Reaktion überschüssigen Phosphortrichlorides mit wasserhaltiger Phosphorsäure, z.B. mit handelsüblicher etwa 75%-iger bis etwa 95%-iger, vorzugsweise etwa 85%-iger Phosphorsäure, in situ gebildet wird. Die Umsetzung wird vorteilhaft unter Erwärmen, z.B. auf etwa 70° bis etwa 120° C, in einem geeigneten Lösungsmittel, wie Tetrachloräthan, Trichloräthan, Chlorbenzol, Chlortoluol oder Paraffinöl, und unter hydrolytischer Aufarbeitung durchgeführt.

Die Ausgangsstoffe der Formel III können, soweit sie nicht bekannt sind, beispielsweise hergestellt werden, indem man eine entsprechende Verbindung der Formel

$R_1 - CH_3$ (IIIa),

mit einer starken Base; beispielsweise einer der unter der Verfahrensvariante a) genannten Metallbasen in das Carbeniatsalz überführt und dieses mit Kohlendioxid oder einer Verbindung der Formel Y-$X_3$ (IIIb), worin Y Halogen, wie Chlor oder Brom bedeutet, z.B. mit einem Carbamylhalogenid, Iminoätherhalogenid oder vor allem einem Halogencyan, wie Chlorcyan, umsetzt.

Für den intermediären Schutz eines substituierbaren N-Atoms des Restes $R_1$ eignen sich die üblichen N-Schutzgruppen und Einführungsund Abspaltungsverfahren derselben, beispielsweise Diniederalkoxymethylgruppen, wie Dimethoxymethyl, die durch Säurebehandlung abgespalten werden können, 2,2,2-Trihalogen-, wie 2,2,2-Trijod-, 2,2,2-Tribrom- oder 2,2,2-Trichloräthoxycarbonylreste, die z.B. durch Behandeln mit Zink in Essigsäure abgespalten werden können, α-Phenylniederalkoxycarbonylreste, wie Carbobenzoxy oder Trityl, die z.B. durch katalytische Hydrierung abgespalten werden können, sowie Niederalkansulfonylgruppen wie Methansulfonyl, die z.B. durch Behandeln mit Bis(2-Methoxyäthoxy)-natriumaluminiumhydrid abgespalten werden können, ebenso aber auch α-Phenylalkyl- oder Alkylgruppen, deren Abspaltung nachstehend behandelt wird.

Verfahrensgemäss oder nach einem anderen an sich bekannten Verfahren erhaltene Verbindungen der Formel 1 können in an sich bekannter Weise in andere Verbindungen der Formel 1 überführt werden.

So kann man beispielsweise Verbindungen der Formel 1, worin $R_2$ Amino ist durch Behandlung mit salpetriger Säure in die entsprechenden Verbindungen I, worin $R_2$ Hydroxy ist, überführen. Die Behandlung mit salpetriger Säure erfolgt in üblicher Weise unter Freisetzung derselben in wässriger Lösung aus einem ihrer Salze, z.B. aus Natriumnitrit, durch Säurebehandlung, z.B. Einwirkung von Salzsäure, wobei intermediär ein entsprechendes, instabiles Diazoniumsalz, z.B. -chlorid, gebildet wird, das unter Einführung der α-Hydroxygruppe Stickstoff abspaltet.

Ferner kann man in Verbindungen der Formel I, worin der Rest $R_1$ durch Niederalkyl oder unsubstituiertes oder durch Niederalkyl, Niederalkoxy und/oder Halogen substituiertes Phenylniederalkyl N-substituiert ist, den N-Substituenten abspalten, Niederalkyl beispielsweise durch Behandeln mit einem Halogenameisensäureester, wie einem Brom- oder Chlorameisensäureniederalkylester, und anschliessende Hydrolyse des gebildeten Carbamates und α-Phenyl-Niederalkylreste beispielsweise durch Hydrogenolyse, z.B. Behandeln mit Wasserstoff in Gegenwart eines Hydrierungs-Katalysators, z.B. von Palladium auf Kohle oder/und Platinoxid oder durch metallische Reduktion, z.B. Behandlung mit einem Alkalimetall in Ammoniak.

Erhaltene freie Verbindungen der Formel I einschliesslich ihrer inneren Salze der Formel I können durch partielle oder vollständige Neutralisation mit einer der eingangs genannten Basen in Basesalze überführt werden. In analoger Weise kann man auch Säureadditionssalze in die entsprechenden freien Verbindungen bzw. deren innere Salze überführen.

Umgekehrt kann man erhaltene freie Verbindungen der Formel I durch Behandlung mit einer der eingangs genannten Protonensäuren in Säureadditionssalze der Formel I" überführen.

Erhaltene Salze können in an sich bekannter Weise in die freien Verbindungen umgewandelt werden, z.B. durch Behandeln mit einem sauren Reagens, wie einer Mineralsäure, bzw. einer Base, z.B. Alkalilauge.

Die Verbindungen einschliesslich ihrer Salze können auch in Form ihrer Hydrate erhalten werden oder das zur Krisallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der Erfindung.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung, welche den pharmakologischen Wirkstoff allein oder zusammen mit einem pharmazeutisch anwendbaren Trägermaterial enthalten.

Die neuen pharmazeutischen Präparate enthalten z.B. von etwa 10 % bis etwa 80 %, vorzugsweise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragéee-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. vom Mais-, Weizen-, Reis- oder Kartoffelstärke, Gelatine, Tragacanth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium-

oder Calciumstearat, und/oder Polyäthylenglykol. Dragéee-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Die vorliegende Erfindung betrifft ebenfalls die Verwendung der Verbindungen der Formeln I und ihrer Salze, vorzugsweise zur Behandlung von Entzündungen, in erster Linie auf Störungen des Calciumstoffwechsels zurückzuführenden Ekrankungen, z.B. des rheumatischen Formenkreises, und besonders von Osteoporeosen.

Dosierungen unter 0,001 mg/kg Körpergewicht beeinflussen die pathologische Verkalkung bzw. die Auflösung von harten Geweben nur unerheblich. Bei Dosierungen von über 100 mg/kg Körpergewicht können langfristig toxische Nebenwirkungen auftreten. Die Verbindungen der Formel I und ihre Salze können sowohl oral als auch hypertonischer Lösung subkutan, intramuskulär oder intravenös appliziert werden. Die bevorzugten Tagesdosen für diese Anwendungen liegen bei oraler Anwendung im Bereich von etwa 0,1 bis 5 mg/kg, bei subkutaner und intramuskulärer Applikation im Bereich von etwa 0,1 bis 1 mg/kg und bei intravenöser Applikation im Bereich von etwa 0,01 bis 2 mg/kg.

Die Dosierung der verwendeten Verbindungen ist jedoch variabel und hängt von den jeweiligen Konditionen wie Art und Schwere der Erkrankung, Dauer der Behandlung und der jeweiligen Verbindung ab. Einzeldosen enthalten beispielsweise von 0,01 bis 10 mg, Dosiseinheitsformen für parenterale, wie intravenöse Applikation z.B. von 0,01 bis 0,1 mg, vorzugsweise 0,02 bis 0,08 mg, orale Dosiseinheitsformen z.B. von 0,2 bis 2,5 mg, vorzugsweise 0,3 bis 1,5 mg pro kg Körpergewicht. Die bevorzugte Einzeldosierung beträgt bei oraler Applikation 10 bis 100 mg und bei intravenöser Applikation 0,5 bis 5 mg. Es können jedoch bis zu 4 Einzeldosen täglich verabreicht werden. Die höheren Dosierungen bei oraler Applikation sind infolge der begrenzten Resorption erforderlich. Bei längerdauernden Behandlungen kann nach anfänglich höherer Dosierung normalerweise auf niedrige Dosierungen umgestellt werden, um den gewünschten Effekt aufrechtzuerhalten.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1:

8,6 g (0,053 Mol) 0,05 Mol (1-Methylimidazol-4-yl)-essigsäure werden mit 7,1 ml 85%-iger Phosphorsäure und 25 ml Chlorbenzol unter Rühren und Rückfluss auf 100° erhitzt. Dann werden bei 100° 13,9 ml Phosphortrichlorid zugetropft, wobei Gasentwicklung stattfindet. Das Reaktionsgemisch scheidet im Lauf von 30 Minuten eine dicke Masse ab. Man erhitzt noch 3 Stunden auf 100° und dekantiert dann das überstehende Chlorbenzol ab. Die zurückbleibende zähe Masse wird mit 40 ml 9-n Chlorwasserstoffsäure 3

Stunden unter Rühren und Rückfluss zum Sieden erhitzt. Man filtriert heiss unter Kohlezusatz und verdünnt das Filtrat mit Aceton, wobei sich die rohe 2-(1-Methylimidazol-4-yl)-1-hydroxy-äthan-1,1-diphosphonsäure abscheidet. Diese wird aus Wasser umkristallisiert, Smp. 261° (Zers.)

Beispiel 2:

19,7 g (0,1 Mol) 1-Benzylimidazol-2-ylacetonitril werden mit 13,4 ml 85%-iger Phosphorsäure und 50 ml Chlorbenzol unter Rühren und Rückfluss auf 100° erhitzt. Dann werden 27 ml Phosphortrichlorid bei 100° zugetropft, wobei Gasentwicklung stattfindet. Das Reaktionsgemisch scheidet im Lauf von 30 Minuten eine dicke Masse ab. Man erhitzt noch 3 Stunden auf 100° und dekantiert dann das überstehende Chlorbenzol ab. Die zurückbleibende zähe Masse wird mit 100 ml 9-n Chlorwasserstoffsäure 3 Stunden unter Rühren und Rückfluss zum Sieden erhitzt. Man filtriert heiss unter Kohlezusatz und kühlt das Filtrat ab, wobei sich die 1-Amino-2-(1-benzylimidazol-2-yl)-äthan-1,1-diphosphonsäure abscheidet.

Beispiel 3:

In analoger Weise wie in Beispiel 2 beschrieben erhält man ausgehend von 0,1 Mol (1-Methylimidazol-4-yl)acetonitril auch 1-Amino-2-(1-methylimidazol-4-yl)äthan-1,1-diphosphonsäure sowie deren Salze, z.B. Dinatriumsalze.

Beispiel 4:

Durch Umsetzung von 1-Methylimidazol-2-ylmethylbromid, Benzylimidazol-2-ylmethylchlorid, Toluolsulfonsäure(imidazol-1-methyl)ester, Imidazol-4-ylmethylchlorid bzw. Thiazolyl-2-ylmethyl-bromid mit Methandiphosphonsäuretetraethylester und Hydrolyse der primär erhaltenen Aethandiphosphonsäureester in Analogie zu Beispiel 7 oder 10, kann man ferner
2-(1-Methylimidazol-2-yl)äthan-1,1-diphosphonsäure,
Smp. 295° (Zers.);
2-(1-Benzylimidazol-2-yl)äthan-1,1-diphosphonsäure-monohydrat;
Smp. 181-183°;
2-(Imidazol-1-yl)äthan-1,1-diphosphonsäure, Smp. 255° (Zers.);
2-(Imidazol-4-yl)äthan-1,1-diphosphonsäure und
2-(Thiazol-2-yl)äthan-1,1-diphosphonsäure, Smp. 259° (Zers.),
und deren Salze, z.B. Dinatriumsalze, herstellen.

Beispiel 5:

In analoger Weise wie in Beispiel 1 beschrieben erhält man ausgehend von 1-Methylimidazol-2-essigsäure-hydrochlorid die 2-(1-Methylimidazol-2-yl)-1-hydroxy-äthan-1,1-diphosphonsäure-monohydrat, Smp. 261° (Zers.).

Das Ausgangsmaterial kann z.B. folgendermassen hergestellt werden: 5,0 g (0,032 Mol) 1-Methyl-2-cyanomethyl-imidazol-hydrochlorid werden mit 15 ml Eisessig und 15 ml 36%-ige Chlorwasserstoffsäure 24 Stunden unter Rückfluss zum Sieden erhitzt. Dann wird unter vermindertem Druck zur Trockne eingedampft, der Rückstand mit 30 ml heissem Eisessig aufgenommen und vom ungelösten Ammoniumchlorid abfiltriert. Das Filtrat wird eingedampft und mit Aceton versetzt. Man erhält das 1-Methyl-2-carboxymethyl-imidazol-hydrochlorid, Smp. 163-164°C (Ausbeute 91 % der Theorie).

Beispiel 6:

In analoger Weise wie in Beispiel 1 beschrieben erhält man ausgehend von
1-Benzylimidazol-2-essigsäure-hydrochlorid bzw.
1-Methylimidazol-2-essigsäure-hydrochlorid die
2-(1-Benzylimidazol-2-yl)-1-hydroxy-äthan-1,1-diphosphonsäure,
Smp. 171° (Zers.), und die
2-(1-Methylimidazol-2-yl)-1-hydroxy-äthan-1,1-diphosphonsäure-mono
hydrat, Smp. 261° (Zers.),
und deren Salze, z.B. Natriumsalze. Das Ausgangsmaterial, 1-Benzylimidazol-2-essigsäure-hydrochlorid, Smp. 124-125°, kann in analoger Weise wie in Beispiel 2 beschrieben hergestellt werden.

Beispiel 7:

14,8 g (0,051 Mol) Methandiphosphonsäuretetraäthylester werden zu einer Suspension von 2,4 g Natriumhydrid in 35 ml absolutem Tetrahydrofuran getropft und bei Raumtemperatur bis zum Ende der Gasentwicklung gerührt. Dann werden 11,3 g (0,0465 Mol) 1-Benzyl-2-chlormethylimidazol-hydrochlorid portionenweise eingetragen. Das Reaktionsgemisch wird unter Rühren und Rückfluss 20 Stunden lang zum Sieden erhitzt. Danach wird vom abgeschiedenen Natriumchlorid abfiltriert und das Filtrat unter vermindertem Druck eingedampft. Man erhält den rohen (1-Benzyl-imidazol-2-yl-methyl)-methan-diphosphonsäuretetraäthylester. 3,0 g (0,065 Mol) 2-(1-Benzylimidazol-2-yl)äthan-1,1-diphosphonsäuretetraäthylester werden mit 12 ml 36%-iger Chlorwasserstoffsäure unter Rückfluss 20 Stunden zum Sieden erhitzt. Nach Eindampfen und Kristallisation des Rückstands aus wässrigem Methanol erhält man das 2-(1-Benzylimidazol-2-yl)-äthan-1,1-diphosphonsäure-monohydrat, Smp. 181-183°. (Ausbeute 30 % der Theorie).

Beispiel 8:

Analog Beispiel 7 erhält man aus
1-Methyl-2-chlormethylimidazol-hydrochlorid und
2-Chlormethylthiazol-hydrochlorid
durch Umsetzung zu den entsprechenden Aethandiphosphonsäuretetraäthylestern und anschliessende Esterspaltung mit Trimethylbromsilan in der beschriebenen Weise:
2-(1-Methylimidazol-2-yl)äthan-1,1-diphosphonsäure, Smp. 295° (Zers.).,
2-(Thiazol-2-yl)äthan-1,1-diphosphonsäure, Smp. 259° (Zers.) sowie deren Salze, z.B. Dinatriumsalze, und Hydrate.

Das Ausgangsmaterial verwendete 1-Methyl-5-chlormethyl-1H-1,2,4-triazol-hydrochlorid kann wie folgt hergestellt werden:
11,1 g (0,10 Mol) 5-Hydroxymethyl-1-methyl-1H-1,2,4-triazol werden in 25 ml Dichlormethan gelöst. Unter Eiskühlung und Rühren werden 29,7 g Thionylchlorid zugetropft. Anschliessend rührt man eine Stunde lang bei Raumtemperatur und danach 20 Minuten bei Siedetemperatur am Rückfluss. Der erhaltene Niederschlag wird abgenutscht, mit Diäthyläther gewaschen und im Vakuum getrocknet. Smp. 136-137° C.

Beispiel 9:

In analoger Weise wie in Beispiel 1 beschrieben erhält man ausgehend von 1-Imidazolessigsäure-hydrochlorid, 1-Pyrazolessigsäure-hydrochlorid bzw. 2-(Imidazol-1-yl)-1-hydroxyäthan-1,1-diphosphonsäure, Smp. 239° (Zers.).

Beispiel 10:

3,3 g (0,0072 Mol) 2-(1-Benzylimidazol-2-yl)äthan-1,1-diphosphonsäure-tetraäthylester werden in 50 ml flüssigem Ammoniak gelöst und allmählich unter Rühren mit 1,0 g Natrium in kleinen Stücken versetzt, bis die blaue Farbe der Lösung längere Zeit bestehen bleibt. Dann werden 2,35 g Ammoniumchlorid portionenweise zugegeben. Man lässt nun den Ammoniak verdampfen, nimmt den Rückstand mit Diäthyläther auf, filtriert und dampft das Filtrat ein. Man erhält so den 2-(Imidazol-2-yl)äthan-1,1-diphosphonsaure-tetraäthylester als farbloses Oel.

2,3 g (0,0062 Mol) 2-(Imidazol-2-yl)äthan-1,1-diphosphonsäure-tetraäthylester werden in 20 ml Methylenchlorid gelöst, mit 4,8 ml Trimethylbromsilan versetzt und 24 Stunden bei Raumtemperatur stehengelassen. Dann wird unter vermindertem Druck eingedampft und der Rückstand mit 10 ml Methanol und 1 ml Wasser versetzt. Man erhält die 2-(Imidazol-2-yl)äthan-1,1-diphosphonsäure, Smp. 279-282° (Zers.).

Beispiel 11:

8,6 g (0,053 Mol) Imidazol-1-ylessigsäure-hydrochlorid werden mit 7,1 ml 85%-iger Phosphorsäure und 25 ml Chlorbenzol unter Rühren und Rückfluss auf 100° erhitzt. Dann werden bei 100° 13,9 ml Phosphortrichlorid zugetropft, wobei Gasentwicklung stattfindet. Das Reaktionsgemisch scheidet im Lauf von 30 Minuten eine dicke Masse ab. Man erhitzt noch 3 Stunden auf 100° und dekantiert dann das überstehende Chlorbenzol ab. Die zurückbleibende zähe Masse wird mit 40 ml 9-n Chlorwasserstoffsäure 3 Stunden unter Rühren und Rückfluss zum Sieden erhitzt. Man filtriert heiss unter Kohlezusatz und verdünnt das Filtrat mit Aceton, wobei siche die rohe 2-(Imidazol-1-yl)-1-hydroxy-äthan-1,1-diphosphonsäure abscheidet. Diese wird aus Wasser umkristallisiert, Smp. 239° (Zers.) (Ausbeute 41 % d. Th.)

Beispiel 12:

Analog Beispiel 10 erhält man ausgehend von 2-(Imidazol-1-yl)äthan-1,1-diphosphonsäuretetraäthylester durch Behandlung mit Trimethylbromsilan und Aufarbeitung mit wässrigem Methanol 2-(Imidazol-1-yl)äthan-1,1-diphosphonsäure, Smp. 255° (Zers.).

Der Ausgangsester kann z.B. folgendermassen hergestellt werden: 0,10 g Natriumhydrid werden in 4,0 ml absolutem Tetrahydrofuran suspendiert. Eine Lösung vom 0,27 g Imidazol (0,04 Mol) in 2,0 ml Tetrahydrofuran wird langsam zugetropft. Die erhaltene klare Reaktionslösung wird mit 1,2 g Vinylidendiphosphonsäuretetraäthylester versetzt und 24 Stunden bei Raumtemperatur aufbewahrt. Dann werden 2 ml 2-n-äthanolische Chlorwasserstoffsäure zugegeben. Das ausgeschiedene Natriumchlorid wird abfiltriert und das Filtrat eingedampft.

Beispiel 13:

In analoger Weise wie in Beispiel 11 erhält man ausgehend von 0,05 Mol 4H-1,2,4-Triazol-4-ylessigsäure 2-(4H-1,2,4-Triazol-4-yl)-1-hydroxy-äthan-1,1-diphosphonsäure sowie deren Salze, z.B. Dinatriumsalze.

Beispiel 14:

Durch Umsetzung von p-Toluolsulfonsäure(imidazol-1-yl-methyl)ester mit Methandiphosphonsäuretetraethylester und Hydrolyse der primär erhaltenen Aethandiphosphonsäureester in Analogie zu Beispiel 5 kann man ferner 2-(Imidazol-1-yl)äthan-1,1-diphosphonsäure, Smp. 255° (Zers.), und deren Salze, z.B. ihr Dinatriumsalz, herstellen.

Beispiel 15:

Analog Beispiel 5 erhält man aus 1-Benzyl-2-cyano-methyl-imidazol das 1-Benzyl-2-carboxymethyl-imidazol-hydrochlorid, Smp. 124-125°.

Analog Beispiel 11 erhält man aus 1-Benzyl-2-carboxymethyl-imidazol-hydrochlorid die 2-(1-Benzylimidazol-2-yl)-1-hydroxy-äthan-1,1-diphosphonsäure, Smp. 171° (Zers.).

Beispiel 16:

3,59 g (0,01 Mol) 1-Amino-2-(1-benzylimidazol-2-yl)äthan-1,1-diphosphonsäure werden in 20 ml 1-n. Natronlauge gelöst, mit 0,82 g Natriumnitrit versetzt und auf 0° abgekühlt. Dann tropft man unter Rühren langsam 18 ml 2-n. Chlorwasserstoffsäure zu. Anschliessend rührt man noch eine Stunde bei 0-10° und filtriert das ausgeschiedene Produkt. Nach Umkristallisation aus Wasser erhält man die 2-(1-Benzylimidazol-2-yl)-1-hydroxy-äthan-1,1-diphosphonsäure, Smp. 171° (Zers.), Ausbeute 47 %.

Beispiel 17:

In analoger Weise wie in den Beispielen 1 bis 16 beschrieben, kann man ferner
2-[2-Phenylimidazol-4(5)-yl]äthan-1,1-diphosphonsäure;
2-(4,5-Dimethylimidazol-1-yl)-1-hydroxy-äthan-1,1-diphosphonsäure und
2-(2-Methylimidazol-1-yl)-1-hydroxy-äthan-1,1-diphosphonsäure, Smp. 245-246°,
und deren Salze, z.B. Dinatriumsalze, herstellen.

Beispiel 18:

Tabletten, enthaltend 100 mg Wirkstoff, z.B. 2-(Imidazol-1-yl)-1-hydroxy-äthan-1,1-diphosphonsäure oder ein Salz, z.B. das Dinatriumsalz, davon, können folgendermassen hergestellt werden:

| Bestandteile (für 1000 Tabletten) | |
|---|---|
| Wirkstoff | 100,0 g |
| Lactose | 100,0 g |
| Weizenstärke | 47,0 g |
| Magnesiumstearat | 3,0 g |

Herstellung:

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 100 mg einer anderen der in den Beispielen 1-17 genannten Verbindungen der Formel I hergestellt werden, wobei diese auch in Form von Salzen und Basen, z.B. als Natriumsalz, vorliegen können.

Beispiel 19:

Kautabletten, enthaltend 75 mg Wirkstoff, z.B. 2-(Imidazol-1-yl)-1-hydroxy-äthan-1,1-diphosphonsäure oder ein Salz, z.B. das Dinatriumsalz davon, können z.B. folgendermassen hergestellt werden:

| Zusammensetzung: (für 1000 Tabletten) | |
|---|---|
| Wirkstoff | 75,0 g |
| Mannit | 230,0 g |
| Lactose | 100,0 g |
| Talkum | 21,0 g |
| Glycin | 12,5 g |
| Stearinsäure | 10,0 g |
| Saccharin | 1,5 g |
| 5 %-ige Gelatinelösung | q.s. |

Herstellung:

Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

In analoger Weise können auch Kautabletten, enthaltend jeweils 75 mg einer anderen der in den Beispielen 1-17 genannten Verbindungen der Formel I hergestellt werden, wobei diese auch in Form von Salzen mit Basen, z.B. als Natriumsalz, vorliegen können.

Beispiel 20:

Tabletten, enthaltend 10 mg Wirkstoff, z.B. 2-(Imidazol-1-yl)-hydroxy-äthan-1,1-diphosphonsäure oder ein Salz, z.B. das Dinatriumsalz davon, können folgendermassen hergestellt werden:

| Zusammensetzung (für 1000 Tabletten) | |
|---|---|
| Wirkstoff | 10,0 g |
| Lactose | 115,7 g |
| Maisstärke | 17,5 g |
| Polyethylenglykol 6000 | 5,0 g |
| Talkum | 5,0 g |
| Magnesiumstearat | 4,0 g |
| entmineralisiertes Wasser | q.s. |

Herstellung:

Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend 10 mg einer anderen Verbindung der Formel I gemäss den Beispielen 1-17 hergestellt werden, wobei diese auch in Form von Salzen mit Basen, z.B. als Natriumsalz, vorliegen können.

Beispiel 21:

Gelatinesteckkapseln enthaltend 100 mg Wirkstoff, z.B. 2-(Imidazol-4-yl)-1-hydroxy-äthan-1,1-diphosphonsäure oder ein Salz, z.B. das Dinatriumsalz davon, können folgendermassen hergestellt werden:

| Zusammensetzung (für 1000 Kapseln) | |
|---|---|
| Wirkstoff | 350,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem Wirkstoff (lyophilisiert) gesiebt und beide Komponenten 10 Minuten lang innig vermischt. Dann wird die mikrokristalline Cellulose durch ein Sieb mit einer Maschenweite von 0,9 mm hinzugesiebt und erneut 10 Minuten innig vermischt. Zuletzt wird das Magnesiumstearat durch ein Sieb mit einer Maschenweite von 0,8 mm hinzugesiebt und nach 3 Minuten weiteren Mischens je 390 mg der Mischung in Gelatinesteckkapseln der Grösse 0 (elongated) abgefüllt.

In analoger Weise können auch Kapseln, enthaltend 100 mg einer anderen Verbindung der Formel I gemäss den Beispielen 1 bis 17 hergestellt werden, wobei diese auch in Form von Salzen mit Basen, z.B. als Dinatriumsalz, vorliegen können.

Beispiel 22:

Eine 0,2 %ige Injektions- bzw. Infusionslösung kann beispielsweise folgendermassen hergestellt werden.

Wirkstoff, z.B. 2-(Imidazol-1-yl)-1-hydroxy-äthan-1,1-diphosphonsäure oder ein Salz,

| | |
|---|---|
| z.B. das Natriumsalz davon | 5,0 g |
| Natriumchlorid | 22,5 g |
| Phosphatpuffer pH = 7,4 | 300,0 g |
| Wasser, entmin. | ad 2500,0 ml |

Der Wirkstoff wird in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen (enthaltend je 2,0 bzw. 5,0 mg Wirkstoff abgefüllt.

**Patentansprüche**
**Patentansprüche für folgende Vertragsstaaten : AT, BE, CH, DE, FR, GB, IT, LI, LU, NL, SE**

1. Substituierte Alkandiphosphonsäuren, insbesondere Heteroarylalkandiphosphonsäuren der Formel

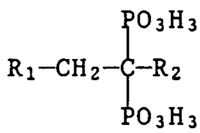

worin $R_1$ einen Imidazolyl-, 2H-1,2,3-, 1H-1,2,4- oder 4H-1,2,4-Triazolyl-, Tetrazolyl-, Oxazolyl-, Isoxazolyl-, Oxadiazolyl-, Thiazolyl- oder Thiadiazolylrest bedeutet, der unsubstituiert durch Niederalkyl, durch Niederalkoxy, durch Phenyl, welches seinerseits durch Niederalkyl, Niederalkoxy und/oder Halogen mono- oder disubstituiert sein kann, durch Hydroxy, durch Diniederalkylamino, durch Niederalkylthio und/oder durch Halogen C-mono- oder disubstituiert und an einem substituierbaren N-Atom durch Niederalkyl oder durch Phenylniederalkyl, welches seinerseits im Phenylteil durch Niederalkyl, Niederalkoxy und/oder Halogen mono- oder disubstituiert sein kann, N-substituiert ist, alk Niederalkylen bedeutet und $R_2$ Wasserstoff, Hydroxy, Amino, Niederalkylthio oder Halogen bedeutet, wobei nieder Reste bis und mit 7 C-Atome aufweisen, mit den Massgaben, dass $R_1$ von Imidazol-4-yl, 2-Methylthiazol-4-yl, 2-Methylthiazol-5-yl und 1,2,5-Thiadiazol-4-yl verschieden ist, wenn $R_2$ Wasserstoff darstellt, dass $R_1$ von Imidazol-2-yl, 5(4)-Methylimidazol-4(5)-yl, 2H-1,2,3-Triazol-4-yl, 1H-1,2,4-Triazol-1-yl, 1H-1,2,4-Triazol-3-yl, 5-Aethoxy-, 5-Aethoxy-2-methyl-, 2-Chlor-, 5-Methoxy- und 2,5-Dimethyloxazol-4-yl, 3-Methyl- und 3-Phenylisoxazol-5-yl, 3-Methyl-1,2,5-oxadiazol-4-yl,2-Methyl-1,3,4-oxadiazol-5-yl, 3-Phenyl-1,2,4-oxadiazol-5-yl, 2-Chlor- und 2-Methylthiazol-5-yl, 1,2,3- und 1,2,5-Thiadiazol-4-yl, 1,2,3-Thiadiazol-5-yl, 4-Methyl-1,2,3-thiadiazol-5-yl und 3-Methyl- und 3-Phenyl-1,2,4-thiadiazol-5-yl verschieden ist, wenn $R_2$ Hydroxy darstellt, und dass $R_1$ von Imidazol-4-yl verschieden ist, wenn $R_2$ Amino oder Dimethylamino darstellt, und ihre Salze.

2. Verbindungen der Formel I, worin $R_1$ einen unsubstituierten oder durch Niederalkyl, Niederalkoxy, unsubstituiertes oder durch Niederalkyl, Niederalkoxy und/oder Halogen mono- oder disubstituiertes Phenyl, Hydroxy, Diniederalkylamino, Niederalkylthio und/oder Halogen C-mono- oder disubstituierten und/oder an einem substituierbaren N-Atom durch Niederalkyl oder unsubstituiertes oder durch Niederalkyl, Niederalkoxy und/oder Halogen mono- oder disubstituiertes Phenylniederalkyl N-substituierten Imidazolyl-, 2H-1,2,3-oder 4H-1,2,4-Triazolyl-, Tetrazolyl-, Oxazolyl-, Isoxazolyl-, Oxadiazolyl-, Thiazolyl- oder Thiadiazolylrest bedeutet und $R_2$ Wasserstoff, Hydroxy, Amino, Niederalkylthio oder Halogen bedeutet, und ihre Salze.

3. Verbindungen der Formel I, worin $R_1$ einen unsubstituierten oder durch $C_1$-$C_4$-Alkyl, $C_1$-$C_4$-Alkoxy, Phenyl, Hydroxy, Di-$C_1$-$C_4$-Alkylamino, $C_1$-$C_4$-Alkylthio und/oder Halogen der Atomnummer bis und mit 35 C-mono- oder di-substituierten und/oder durch $C_1$-$C_4$-Alkyl oder Phenyl-$C_1$-$C_4$-alkyl N-substituierten Imidazolyl-, 4H-1,2,4-Triazol-4-yl- oder Thiazolylrest bedeutet und $R_2$ Hydroxy, Wasserstoff oder Amino bedeutet, und ihre Salze.

4. Verbindungen der Formel I, worin $R_1$ einen unsubstituierten oder durch Phenyl C-substituierten bzw. durch $C_1$-$C_4$-Alkyl, C- oder N-substituierten Imidazol-2- oder -4-ylrest, einen unsubstituierten Thiazolylrest oder einen unsubstituierten oder durch $C_1$-$C_4$-Alkyl N-substituierten 1H-1,2,4-Triazolylrest darstellt und $R_2$ Hydroxy oder Wasserstoff bedeutet, und ihre Salze.

**5.** Verbindungen gemäss Anspruch 1, worin $R_1$ Imidazol-1-yl, 2-, 4- oder 5-$C_1$-$C_4$-Alkylimidazol-1-yl, 3-$C_1$-$C_4$-Alkyl-1H-1,2,4-triazol-1-yl, 4H-Triazol-4-yl, 3-$C_1$-$C_4$-Alkyl-4H-1,2,4-triazol-4-yl oder 1H-Tetrazol-1-yl bedeutet und alk sowie $R_2$ die in Anspruch 1 angegebenen Bedeutungen haben, und ihre Salze.

**6.** Verbindungen der Formel I, worin $R_1$ einen unsubstituierten oder durch Phenyl bzw. durch $C_1$-$C_4$-Alkyl C-substituierten Imidazol-1-yl-, 4H-1,2,4-Triazol-4-yl- oder Tetrazol-1-ylrest darstellt und $R_2$ Hydroxy oder Wasserstoff bedeutet, und ihre Salze.

**7.** 2-(1-Methylimidazol-2-yl)-1-hydroxy-äthan-1,1-diphosphonsäure oder ein Salz davon.

**8.** 2-(1-Benzylimidazol-2-yl)-1-hydroxy-äthan-1,1-diphosphonsäure oder ein Salz davon.

**9.** 2-(1-Methylimidazol-4-yl)-1-hydroxy-äthan-1,1-diphosphonsäure oder ein Salz davon.

**10.** 1-Amino-2-(1-methylimidazol-4-yl)äthan-1,1-diphosphonsäure oder ein Salz davon.

**11.** 1-Amino-2-(1-benzylimidazol-4-yl)äthan-1,1-diphosphonsäure oder ein Salz davon.

**12.** 2-(1-Methylimidazol-2-yl)äthan-1,1-diphosphonsäure oder ein Salz davon.

**13.** 2-(1-Benzylimidazol-2-yl)äthan-1,1-diphosphonsäure oder ein Salz davon.

**14.** 2-(Imidazol-1-yl)-1-hydroxy-äthan-1,1-diphosphonsäure oder ein Salz davon.

**15.** 2-(Imidazol-1-yl)äthan-1,1-diphosphonsäure oder ein Salz davon.

**16.** 2-(4H-1,2,4-Triazol-4-yl)-1-hydroxy-äthan-1,1-diphosphonsäure oder ein Salz davon.

**17.** 2-(Thiazol-2-yl)äthan-1,1-diphosphonsäure oder ein Salz davon.

**18.** 2-(Imidazol-2-yl)äthan-1,1-diphosphonsäure oder ein Salz davon.

**19.** 2-[2-Methylimidazol-4(5)-yl]äthan-1,1-diphosphonsäure oder ein Salz davon.

**20.** 2-[2-Phenylimidazol-4(5)-yl]äthan-1,1-diphosphonsäure oder ein Salz davon.

**21.** 2-(4,5-Dimethylimidazol-1-yl)-1-hydroxy-äthan-1,1-diphosphonsäureoder ein Salz davon.

**22.** 2-(2-Methylimidazol-1-yl)-1-hydroxy-äthan-1,1-diphosphonsäure oder ein Salz davon.

**23.** Eine Verbindung gemäss einem der Ansprüche 1, 4, 5 und 17 bis 22 zur Anwendung in einem Verfahren zur therapeutischen Behandlung des menschlichen oder tierischen Körpers, z.B. als den Calciumstoffwechsel regulierendes und/oder antiarthritisches Mittel.

**24.** Eine Verbindung gemäss einem der Ansprüche 2, 3 und 6 - 16 zur Anwendung in einem Verfahren zur therapeutischen Behandlung des menschlichen oder tierischen Körpers, z.B. als den Calciumstoffwechsel regulierendes und/oder antiarthritisches Mittel.

**25.** Pharmazeutische Präparate enthaltend eine Verbindung gemäss einem der Ansprüche 1, 4, 5 und 17 bis 23 neben üblichen pharmazeutischen Hilfsstoffen.

**26.** Pharmazeutische Präparate enthaltend eine Verbindung gemäss einem der Ansprüche 2, 3, 6 bis 16 und 24.

**27.** Verfahren zur Herstellung von Verbindungen gemäss Anspruch 1, und ihrer Salze, dadurch gekennzeichnet, dass man

a) in einer an einem substituierbaren N-Atom des Restes $R_1$ gegebenenfalls intermediär geschützten Verbindung der Formel

$$R_1 - CH_2 - \overset{X_1}{\underset{X_2}{C}} - R_2 \qquad (II),$$

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt oder
b) eine an einem substituierbaren N-Atom des Restes $R_1$ gegebenenfalls intermediär geschützte Verbindung der Formel

$R_1$ - $CH_2$ - $X_3$ (III),

worin $X_3$ eine Carboxy-, Carbamyl-, Iminoäther-, Iminoester- oder Cyanogruppe bedeutet, mit phosphoriger Säure und Phosphortrichlorid umsetzt, wobei ausgehend von Verbindungen der Formel IV, worin $X_3$ eine Carbamyl-, Iminoäther-, Iminoester- Cyanogruppe ist, bei der hydrolytischen Aufarbeitung Verbindungen der Formel I erhalten werden, worin $R_2$ Amino ist, und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

**28.** Verfahren gemäss Anspruch 27, dadurch gekennzeichnet, dass man

a) in einer Verbindung der Formel

$$R_1 - CH_2 - \overset{X_1}{\underset{X_2}{C}} - R_2 \qquad (II),$$

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt oder
b) eine Verbindung der Formel

$R_1$ - $CH_2$ - $X_3$ (III),

worin $X_3$ Carboxy oder Cyano bedeutet, mit phosphoriger Säure und Phosphortrichlorid umsetzt, wobei ausgehend von Verbindungen der Formel III, worin $X_3$ Cyano ist, unter hydrolytischer Aufarbeitung Verbindungen der Formel I erhalten werden, worin $R_2$ Amino ist, und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

**29.** Verwendung einer Verbindung gemäss einem der Ansprüche 1 bis 22 zur Herstellung eines für die Behandlung von Calcium-Stoffwechselerkrankungen geeigneten Arzneimittels.

**Patentansprüche für folgende Vertragsstaaten : ES, GR**

**1.** Verfahren zur Herstellung von Alkandiphosphonsäuren, insbesondere Heteroarylalkandiphosphonsäuren der Formel

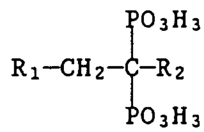

worin $R_1$ einen Imidazolyl-, 2H-1,2,3-, 1H-1,2,4- oder 4H-1,2,4-Triazolyl-, Tetrazolyl-, Oxazolyl-, Isoxazolyl-, Oxadiazolyl-, Thiazolyl-, oder Thiadiazolylrest bedeutet, der unsubstituiert oder durch Niederalkyl, durch Niederalkoxy, durch Phenyl, welches seinerseits durch Niederalkyl, Niederalkoxy und/oder Halogen mono-oder disubstituiert sein kann, durch Hydroxy, durch Diniederalkylamino, durch Niederalkylthio und/oder durch Halogen C-mono-oder disubstituiert und an einem substituierbaren H-Atom durch Niederalkyl oder durch Phenylniederalkyl, welches seinerseits im Phenylteil durch Niederalkyl, Niederalkoxy und/oder Halogen mono-oder disubstituiert sein kann, N-substituiert ist, und $R_2$ Wasserstoff, Hydroxy, Amino, Niederalkylthio oder Halogen bedeutet, mit den Massgaben, dass $R_1$ von Imidazol-4-yl, 2-Methylthiazol-4-yl, 2-Methylthiazol-5-yl und 1,2,5-Thiadiazol-4-yl verschieden ist, wenn $R_2$ Wasserstoff darstellt, dass $R_1$ von Imidazol-2-yl, 5(4)-Methylimidazol-4(5)-yl, 2H-1,2,3-Triazol-4-yl, 1H-1,2,4-Triazol-1-yl, 1H-1,2,4-Triazol-3-yl, 5-Aethoxy-, 5-Aethoxy-2-methyl-, 2-Chlor-, 5-Methoxy- und 2,5-Dimethyloxazol-4-yl, 3-Methyl- und 3-Phenylisoxazol-5-yl, 3-Methyl-1,2,5-oxadiazol-4-yl, 2-Methyl-1,3,4-oxadiazol-5-yl, 3-Phenyl-1,2,4-oxadiazol-5-yl, 2-Chlor- und 2-Methylthiazol-5-yl, 1,2,3- und 1,2,5-Thiadiazol-4-yl, 1,2,3-Thiadiazol-5-yl, 4-Methyl-1,2,3-thiadiazol-5-yl und 3-Methyl- und 3-Phenyl-1,2,4-thiadiazol-5-yl verschieden ist, wenn $R_2$ Hydroxy darstellt, und dass $R_1$ von Imidazol-4-yl verschieden ist, wenn $R_2$ Amino oder Dimethylamino darstellt und ihrer Salze, dadurch gekennzeichnet, dass man

a) in einer an einem substituierbaren H-Atom des Restes $R_1$ gegebenenfalls intermediär geschützten Verbindung der Formel

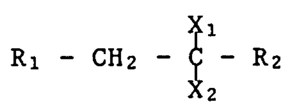 (II),

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt oder

b) eine an einem substituierbaren H-Atom des Restes $R_1$ gegebenenfalls intermediär geschützte Verbindung der Formel

$R_1$ - $CH_2$ - $X_3$ (III),

worin $X_3$ eine Carboxy-, Carbamyl-, Iminoäther-, Iminoester- oder Cyanogruppe bedeutet, mit phosphoriger Säure und Phosphortrichlorid umsetzt, wobei ausgehend von Verbindungen der Formel IV, worin $X_3$ eine Carbamyl-, Iminoäther-, Iminoester- Cyanogruppe ist, bei der hydrolytischen Aufarbeitung Verbindungen der Formel I erhalten werden, worin $R_2$ Amino ist, und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

**2.** Verfahren gemäss Anspruch 1 zur Herstellung von Verbindungen der Formel I, worin $R_1$ einen unsubstituierten oder durch Niederalkyl, Niederalkoxy, unsubstituiertes oder durch Niederalkyl, Niederalkoxy und/oder Halogen mono- oder disubstituiertes Phenyl, Hydroxy, Diniederalkylamino, Niederalkylthio und/oder Halogen C-mono- oder disubstituierten und/oder an einem substituierbaren H-Atom durch Niederalkyl oder unsubstituiertes oder durch Niederalkyl, Niederalkoxy und/oder Halogen mono- oder disubstituiertes Phenylniederalkyl N-substituierten Imidazolyl-, 2H-1,2,3- oder 4H-1,2,4-Triazolyl-, Tetrazolyl-, Oxazolyl-, Isoxazolyl-, Oxadiazolyl-, Thiazolyl-, oder Thiadiazolylrest bedeutet und $R_2$ Wasserstoff, Hydroxy, Amino, Niederalkylthio oder Halogen bedeutet, und ihrer Salze.

**3.** Verfahren gemäss Anspruch 1 zur Herstellung von Verbindungen der Formel I, worin $R_1$ einen

unsubstituierten oder durch $C_1$-$C_4$-Alkyl, $C_1$-$C_4$-Alkoxy, Phenyl, Hydroxy, Di-$C_1$-$C_4$-Alkylamino, $C_1$-$C_4$-Alkylthio und/oder Halogen der Atomnummer bis und mit 35 C- mono- oder di-substituierten und/oder durch $C_1$-$C_4$-Alkyl oder Phenyl-$C_1$-$C_4$-alkyl N-substituierten Imidazolyl-, 4H-1,2,4-Triazolyl- oder Thiazolylrest bedeutet und $R_2$ Hydroxy, Wasserstoff oder Amino bedeutet, und ihrer Salze.

**4.** Verfahren gemäss Anspruch 1 zur Herstellung von Verbindungen der Formel I, worin $R_1$ einen unsubstituierten oder durch Phenyl C-substituierten bzw. durch $C_1$-$C_4$-Alkyl, C- oder N-substituierten Imidazol-2- oder -4-ylrest, einen unsubstituierten Thiazolylrest oder einen unsubstituierten oder durch $C_1$-$C_4$-Alkyl N-substituierten 1H-1,2,4-Triazolylrest darstellt und $R_2$ Hydroxy oder in zweiter Linie Wasserstoff bedeutet, und ihrer Salze.

**5.** Verfahren gemäss Anspruch 1 zur Herstellung von Verbindungen der Formel I, worin $R_1$ einen unsubstituierten oder durch Phenyl bzw. durch $C_1$-$C_4$-Alkyl, C-substituierten Imidazol-1-yl-, 1H-1,2,4-Triazol-1-yl-, 4H-1,2,4-Triazol-4-yl-oder Tetrazol-1-ylrest darstellt und $R_2$ Hydroxy oder in zweiter Linie Wasserstoff bedeutet, und ihrer Salze.

**6.** Verfahren gemäss Anspruch 1 zur Herstellung von Verbindungen der Formel I, worin $R_1$ Imidazol-1-yl, 2-, 4- oder 5-$C_1$-$C_4$-Alkylimidazol-1-yl, 3-$C_1$-$C_4$-Alkyl-1H-1,2,4-triazol-1-yl, 4H-Triazol-4-yl, 3-$C_1$-$C_4$-Alkyl-4H-1,2,4-triazol-4-yl oder 1H-Tetrazol-1-yl bedeutet und alk sowie $R_2$ die in Anspruch 1 angegebenen Bedeutungen haben, und ihrer Salze.

**7.** Verfahren gemäss Anspruch 1 zur Herstellung von Verbindungen der Formel I, worin $R_1$ einen unsubstituierten oder durch $C_1$-$C_4$-Alkyl substituierten Imidazolrest darstellt und $R_2$ Hydroxy oder Wasserstoff bedeutet, und ihrer Salze.

**8.** Verfahren gemäss Anspruch 1 zur Herstellung von 2-(1-Methylimidazol-2-yl)-1-hydroxy-äthan-1,1-diphosphonsäure oder eines ihrer Salze.

**9.** Verfahren gemäss Anspruch 1 zur Herstellung von 2-(1-Benzylimidazol-2-yl)-1-hydroxy-äthan-1,1-diphosphonsäure oder eines ihrer Salze.

**10.** Verfahren gemäss Anspruch 1 zur Herstellung von 2-(1-Methylimidazol-4-yl)-1-hydroxy-äthan-1,1-diphosphonsäure oder eines ihrer Salze.

**11.** Verfahren gemäss Anspruch 1 zur Herstellung von 1-Amino-2-(1-methylimidazol-4-yl)äthan-1,1-diphosphonsäure oder eines ihrer Salze.

**12.** Verfahren gemäss Anspruch 1 zur Herstellung von 1-Amino-2-(1-benzylimidazol-4-yl)äthan-1,1-diphosphonsäure oder eines ihrer Salze.

**13.** Verfahren gemäss Anspruch 1 zur Herstellung von 2-(1-Methylimidazol-2-yl)äthan-1,1-diphosphonsäure oder eines ihrer Salze

**14.** Verfahren gemäss Anspruch 1 zur Herstellung von 2-(1-Benzylimidazol-2-yl)äthan-1,1-diphosphonsäure oder eines ihrer Salze.

**15.** Verfahren gemäss Anspruch 1 zur Herstellung von 2-(Imidazol-1-yl)-1-hydroxy-äthan-1,1-diphosphonsäure oder eines ihrer Salze.

**16.** Verfahren gemäss Anspruch 1 zur Herstellung von 2-(Imidazol-1-yl)äthan-1,1-diphosphonsäure oder eines ihrer Salze.

**17.** Verfahren gemäss Anspruch 1 zur Herstellung von 2-(Thiazol-2-yl)äthan-1,1-diphosphonsäure oder eines ihrer Salze.

**18.** Verfahren gemäss Anspruch 1 zur Herstellung von 2-(4H-1,2,4-Triazol-4-yl)-1-hydroxy-äthan-1,1-diphosphonsäure oder eines ihrer Salze.

**19.** Verfahren gemäss Anspruch 1 zur Herstellung von 2-(Imidazol-2-yl)äthan-1,1-diphosphonsäure oder eines ihrer Salze.

**20.** Verfahren gemäss Anspruch 1 zur Herstellung von 2-(1-Methyl-1H-1,2,4-triazol-5-yl)äthan-1,1-diphosphonsäure oder eines ihrer Salze.

**21.** Verfahren gemäss Anspruch 1 zur Herstellung von 2-[2-Methylimidazol-4(5)-yl]äthan-1,1-diphosphonsäure oder eines ihrer Salze.

**22.** Verfahren gemäss Anspruch 1 zur Herstellung von 2-[2-Phenylimidazol-4(5)-yl]äthan-1,1-diphosphonsäure oder eines ihrer Salze.

**23.** Verfahren gemäss Anspruch 1 zur Herstellung von 2-(4,5-Dimethylimidazol-1-yl)-1-hydroxy-äthan-1,1-diphosphonsäure oder eines ihrer Salze.

**24.** Verfahren gemäss Anspruch 1 zur Herstellung von 2-(2-Methylimidazol-1-yl)-1-hydroxy-äthan-1,1-diphosphonsäure oder eines ihrer Salze.

**25.** Verfahren zur Herstellung von pharmazeutischen Präparaten, dadurch gekennzeichnet, dass man eine Verbindung, erhältlich gemäss einem der Ansprüche 1 bis 24 mit üblichen pharmazeutischen Hilfsstoffen vermischt.

**Claims**

**Claims for the following Contracting States : AT, BE, CH, DE, FR, GB, IT, LU, NL, SE**

**1.** A substituted alkanediphosphonic acid, especially a heteroarylalkanediphosphonic acid of formula

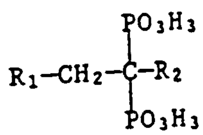

wherein $R_1$ is an imidazolyl, 2H-1,2,3-, 1H-1,2,4- or 4H-1,2,4-triazolyl, tetrazolyl, oxazolyl, isoxazolyl, oxadiazolyl, thiazolyl or thiadiazolyl radical which is unsubstituted or C-mono- or di-substituted by lower alkyl, by lower alkoxy, by phenyl which may in turn be mono- or di-substituted by lower alkyl, lower alkoxy and/or halogen, by hydroxy, by di-lower alkylamino, by lower alkylthio and/or by halogen and is N-substituted at a substitutable N-atom by lower alkyl or by phenyl-lower alkyl which may in turn be mono- or di-substituted in the phenyl moiety by lower alkyl, lower alkoxy and/or halogen, alk is lower alkylene, and $R_2$ is hydrogen, hydroxy, amino, lower alkylthio or halogen, lower radicals having up to and including 7 C-atoms, with the provisos that $R_1$ is other than imidazol-4-yl, 2-methylthiazol-4-yl, 2-methylthiazol-5-yl and 1,2,5-thiadiazol-4-yl when $R_2$ is hydrogen, that $R_1$ is other than imidazol-2-yl, 5-(4)-methylimidazol-4(5)-yl, 2H-1,2,3-triazol-4-yl, 1H-1,2,4-triazol-1-yl, 1H-1,2,4-triazol-3-yl, 5-ethoxy-, 5-ethoxy-2-methyl-, 2-chloro-, 5-methoxy- and 2,5-dimethyl-oxazol-4-yl, 3-methyl- and 3-phenyl-isoxazol-5-yl, 3-methyl-1,2,5-oxadiazol-4-yl, 2-methyl-1,3,4-oxadiazol-5-yl, 3-phenyl-1,2,4-oxadiazol-5-yl, 2-chloro- and 2-methyl-thiazol-5-yl, 1,2,3- and 1,2,5-thiadiazol-4-yl, 1,2,3-thiadiazol-5-yl, 4-methyl-1,2,3-thiadiazol-5-yl and 3-methyl- and 3-phenyl-1,2,4-thiadiazol-5-yl when $R_2$ is hydroxy, and that $R_1$ is other than imidazol-4-yl when $R_2$ is amino or dimethylamino, or a salt thereof.

**2.** A compound of formula I wherein $R_1$ is an imidazolyl, 2H-1,2,3-triazolyl or 4H-1,2,4-triazolyl, tetrazolyl, oxazolyl, isoxazolyl, oxadiazolyl, thiazolyl or thiadiazolyl radical which is unsubstituted or C-mono- or di-substituted by lower alkyl, lower alkoxy, phenyl which is unsubstituted or mono- or di-substituted by lower alkyl, lower alkoxy and/or halogen, hydroxy, di-lower alkylamino, lower alkylthio and/or halogen, and/or is N-substituted at a substitutable N-atom by lower alkyl or phenyl-lower alkyl which is unsubstituted or mono- or di-substituted by lower alkyl, lower alkoxy and/or halogen, and $R_2$ is hydrogen, hydroxy, amino, lower alkylthio or halogen, or a salt thereof.

**3.** A compound of formula I wherein $R_1$ is an imidazolyl, 4H-1,2,4-triazol-4-yl or thiazolyl radical which is unsubstituted or C-mono- or di-substituted by $C_1$-$C_4$alkyl, $C_1$-$C_4$alkoxy, phenyl, hydroxy, di-$C_1$-$C_4$-alkylamino, $C_1$-$C_4$alkylthio and/or halogen having an atomic number of up to and including 35, and/or is N-substituted by $C_1$-$C_4$alkyl or phenyl-$C_1$-$C_4$alkyl, and $R_2$ is hydroxy, hydrogen or amino, or a salt thereof.

**4.** A compound of formula I wherein $R_1$ is an imidazol-2-yl or imidazol-4-yl radical which is unsubstituted or C-substituted by phenyl or C- or N-substituted by $C_1$-$C_4$-alkyl, or is an unsubstituted thiazolyl radical or a 1H-1,2,4-triazolyl radical which is unsubstituted or N-substituted by $C_1$-$C_4$alkyl, and $R_2$ is hydroxy or hydrogen, or a salt thereof.

**5.** A compound according to claim 1 wherein $R_1$ is imidazol-1-yl, 2-, 4- or 5-$C_1$-$C_4$alkylimidazol-1-yl, 3-$C_1$-$C_4$alkyl-1H-1,2,4-triazol-1-yl, 4H-triazol-4-yl, 3-$C_1$-$C_4$alkyl-4H-1,2,4-triazol-4-yl or 1H-tetrazol-1-yl, and alk and $R_2$ are as defined in claim 1, or a salt thereof.

**6.** A compound of formula I wherein $R_1$ is an imidazol-1-yl, 4H-1,2,4-triazol-4-yl or tetrazol-1-yl radical which is unsubstituted or C-substituted by phenyl or by $C_1$-$C_4$-alkyl, and $R_2$ is hydroxy or hydrogen, or a salt thereof.

**7.** 2-(1-Methylimidazol-2-yl)-1-hydroxyethane-1,1-diphosphonic acid or a salt thereof.

**8.** 2-(1-Benzylimidazol-2yl)-1-hydroxyethane-1,1-diphosphonic acid or a salt thereof.

**9.** 2-(1-Methylimidazol-4-yl)-1-hydroxyethane-1,1-diphosphonic acid or a salt thereof.

**10.** 1-Amino-2-(1-methylimidazol-4-yl)ethane-1,1-diphosphonic acid or a salt thereof.

**11.** 1-Amino-2-(1-bensylimidazol-4-yl)ethane-1,1-diphosphonic acid or a salt thereof.

**12.** 2-(1-Methylimidazol-2-yl)ethane-1,1-diphosphonic acid or a salt thereof.

**13.** 2-(1-Benzylimidazol-2yl)ethane-1,1-diphosphonic acid or a salt thereof.

**14.** 2-(Imidazol-1-yl)-1-hydroxyethane-1,1-diphosphonic acid or a salt thereof.

**15.** 2-(Imidazol-1-yl)ethane-1,1-diphosphonic acid or a salt thereof.

**16.** 2-(4H-1,2,4-triazol-4-yl)-1-hydroxyethane-1,1-diphosphonic acid or a salt thereof.

**17.** 2-(Thiazol-2-yl)ethane-1,1-diphosphonic acid or a salt thereof.

**18.** 2-(Imidazol-2-yl)ethane-1,1-diphosphonic acid or a salt thereof.

**19.** 2-[2-Methylimidazol-4(5)-yl]ethane-1,1-diphosphonic acid or a salt thereof.

**20.** 2-[2-Phenylimidazol-4(5)-yl]ethane-1,1-diphosphonic acid or a salt thereof.

**21.** 2-(4,5-Dimethylimidazol-1-yl)-1-hydroxyethane-1,1-diphosphonic acid or a salt thereof.

**22.** 2-(2-Methylimidazol-1-yl)-1-hydroxyethane-1,1-diphosphonic acid or a salt thereof.

**23.** A compound according to any one of claims 1, 4, 5 and 17 to 22 for use in a method for the therapeutic treatment of the human or animal body, for example as an agent that regulates calcium metabolism and/or as an anti-arthritic agent.

**24.** A compound according to any one of claims 2, 3 and 6 to 16 for use in a method for the therapeutic treatment of the human or animal body, for example as an agent that regulates calcium metabolism and/or as an anti-arthritic agent.

**25.** A pharmaceutical composition comprising a compound according to any one of claims 1, 4, 5 and 17 to 23, together with conventional pharmaceutical excipients.

**26.** A pharmaceutical composition comprising a compound according to any one of claims 2, 3, 6 to 16 and 24.

**27.** A process for the preparation of a compound according to claim 1 or a salt thereof, which comprises
a) in a compound of formula

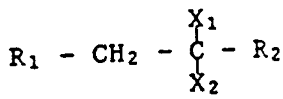

(II),

wherein $X_1$ is a functionally modified phosphono group and $X_2$ is a free or functionally modified phosphono group, which compound may be temporarily protected at a substitutable N-atom of the radical $R_1$, converting $X_1$ and, if appropriate, $X_2$ into the free phosphono group, or
b) reacting a compound of formula

$R_1 - CH_2 - X_3$ (III),

wherein $X_3$ is a carboxy, carbamoyl, imino ether, imino ester or cyano group, which compound may be temporarily protected at a substitutable N-atom of the radical $R_1$, with phosphorous acid and phosphorus trichloride, and, where a start is made from a compound of formula IV wherein $X_3$ is a carbamoyl, imino ether, imino ester or cyano group, there is obtained, upon working up by hydrolysis, a compound of formula I wherein $R_2$ is amino, and, if desired, converting a resultant compound into another compound of formula I and/or a resultant free compound into a salt or a resultant salt into the free compound or into another salt.

**28.** A process according to claim 27, which comprises
a) in a compound of formula

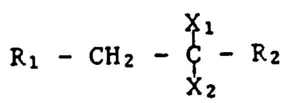

(II),

wherein $X_1$ is a functionally modified phosphono group and $X_2$ is a free or functionally modified phosphono group, converting $X_1$ and, if appropriate, $X_2$ into the free phosphono group, or
b) reacting a compound of formula

$R_1 - CH - X_3$ (III),

wherein $X_3$ is carboxy or cyano, with phosphorous acid and phosphorus trichloride, and, where a start is made from a compound of formula III wherein $X_3$ is cyano, there is obtained, with working up by hydrolysis, a compound of formula I wherein $R_2$ is amino, and, if desired, converting a resultant compound into another compound of formula I and/or a resultant free compound into a salt or a resultant salt into the free compound or into another salt.

**29.** The use of a compound according to any one of claims 1 to 22 for the manufacture of a medicament suitable for the treatment of diseases associated with impaired calcium metabolism.

**Claims for the following Contracting States : ES, GR**

**1.** A process for the preparation of an alkanediphosphonic acid, especially a heteroarylalkanediphosphonic acid of formula

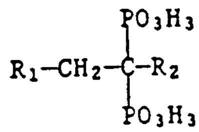

wherein $R_1$ is an imidazolyl, 2H-1,2,3-, 1H-1,2,4- or 4H-1,2,4-triazolyl, tetrazolyl, oxazolyl, isoxazolyl, oxadiazolyl, thiazolyl or thiadiazolyl radical which is unsubstituted or C-mono- or di-substituted by lower alkyl, by lower alkoxy, by phenyl which may in turn be mono- or di-substituted by lower alkyl, lower alkoxy and/or halogen, by hydroxy, by di-lower alkylamino, by lower alkylthio and/or by halogen and is N-substituted at a substitutable N-atom by lower alkyl or by phenyl-lower alkyl which may in turn be mono- or di-substituted in the phenyl moiety by lower alkyl, lower alkoxy and/or halogen, and $R_2$ is hydrogen, hydroxy, amino, lower alkylthio or halogen, with the provisos that $R_1$ is other than imidazol-4-yl, 2-methylthiazol-4-yl, 2-methylthiazol-5-yl and 1,2,5-thiadiazol-4-yl when $R_2$ is hydrogen, that $R_1$ is other than imidazol-2-yl, 5(4)-methylimidazol-4(5)-yl, 2H-1,2,3-triazol-4-yl, 1H-1,2,4-triazol-1-yl, 1H-1,2,4-triazol-3-yl, 5-ethoxy-, 5-ethoxy-2-methyl-, 2-chloro-, 5-methoxy- and 2,5-dimethyl-oxazol-4-yl, 3-methyl- and 3-phenyl-isoxazol-5-yl, 3-methyl-1,2,5-oxadiazol-4-yl, 2-methyl-1,3,4-oxadiazol-5-yl, 3-phenyl-1,2,4-oxadiazol-5-yl, 2-chloro- and 2-methylthiazol-5-yl, 1,2,3- and 1,2,5-thiadiazol-4-yl, 1,2,3-thiadiazol-5-yl, 4methyl-1,2,3-thiadiazol-5-yl and 3-methyl- and 3-phenyl-1,2,4-thiadiazol-5-yl when $R_2$ is hydroxy, and that $R_1$ is other than imidazol-4-yl when $R_2$ is amino or dimethylamino, or a salt thereof, which process comprises

a) in a compound of formula

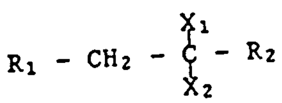

(II),

wherein $X_1$ is a functionally modified phosphono group and $X_2$ is a free or functionally modified phosphono group, which compound may be temporarily protected at a substitutable N-atom of the radical $R_1$, converting $X_1$ and, if appropriate, $X_2$ into the free phosphono group, or

b) reacting a compound of formula

$R_1 - CH_2 - X_3$ (III),

wherein $X_3$ is a carboxy, carbamoyl, imino ether, imino ester or cyano group, which compound may be temporarily protected at a substitutable N-atom of the radical $R_1$, with phosphorous acid and phosphorus trichloride, and, where a start is made from a compound of formula IV wherein $X_3$ is a carbamoyl, imino ether, imino ester or cyano group, there is obtained, upon working up by hydrolysis, a compound of formula I wherein $R_2$ is amino, and, if desired, converting a resultant compound into another compound of formula I and/or a resultant free compound into a salt or a resultant salt into the free compound or into another salt.

**2.** A process according to claim 1 for the preparation of a compound of formula I wherein $R_1$ is an imidazolyl, 2H-1,2,3-triazolyl or 4H-1,2,4-triazolyl, tetrazolyl, oxazolyl, isoxazolyl, oxadiazolyl, thiazolyl or thiadiazolyl radical which is unsubstituted or C-mono- or di-substituted by lower alkyl, lower alkoxy, phenyl which is unsubstituted or mono- or di-substituted by lower alkyl, lower alkoxy and/or halogen, hydroxy, di-lower alkylamino, lower alkylthio and/or halogen, and/or is N-substituted at a substitutable N-atom by lower alkyl or phenyl-lower alkyl which is unsubstituted or mono- or di-substituted by lower alkyl, lower alkoxy and/or halogen, and $R_2$ is hydrogen, hydroxy, amino, lower alkylthio or halogen, or a salt thereof.

**3.** A process according to claim 1 for the preparation of a compound of formula I wherein $R_1$ is an imidazolyl, 4H-1,2,4-triazolyl or thiazolyl radical which is unsubstituted or C-mono- or di-substituted by $C_1$-$C_4$alkyl, $C_1$-$C_4$alkoxy, phenyl, hydroxy, di-$C_1$-$C_4$alkylamino, $C_1$-$C_4$alkylthio and/or halogen having an atomic number of up to and including 35, and/or is N-substituted by $C_1$-$C_4$alkyl or phenyl-$C_1$-$C_4$alkyl,

and $R_2$ is hydroxy, hydrogen or amino, or a salt thereof.

**4.** A process according to claim 1 for the preparation of a compound of formula I wherein $R_1$ is an imidazol-2-yl or imidazol-4-yl radical which is unsubstituted or C-substituted by phenyl or C- or N-substituted by $C_1$-$C_4$alkyl, or is an unsubstituted thiazolyl radical or a 1H-1,2,4-triazolyl radical which is unsubstituted or N-substituted by $C_1$-$C_4$alkyl, and $R_2$ is hydroxy or, less preferably, hydrogen, or a salt thereof.

**5.** A process according to claim 1 for the preparation of a compound of formula I wherein $R_1$ is an imidazol-1-yl, 1H-1,2,4-triazol-1-yl, 4H-1,2,4-triazol-4-yl or tetrazol-1-yl radical which is unsubstituted or C-substituted by phenyl or by $C_1$-$C_4$alkyl, and $R_2$ is hydroxy or, less preferably, hydrogen, or a salt thereof.

**6.** A process according to claim 1 for the preparation of a compound of formula I wherein $R_1$ is imidazol-1-yl, 2-, 4- or 5-$C_1$-$C_4$alkylimidazol-1-yl, 3-$C_1$-$C_4$alkyl-1H-1,2,4-triazol-1-yl, 4H-triazol-4-yl, 3-$C_1$-$C_4$alkyl-4H-1,2,4-triazol-4-yl or 1H-tetrazol-1-yl, and alk and $R_2$ are as defined in claim 1, or a salt thereof.

**7.** A process according to claim 1 for the preparation of a compound of formula I wherein $R_1$ is an imidazole radical which is unsubstituted or substituted by $C_1$-$C_4$-alkyl, and $R_2$ is hydroxy or hydrogen, or a salt thereof.

**8.** A process according to claim 1 for the preparation of 2-(1-methylimidazol-2-yl)-1-hydroxyethane-1,1-diphosphonic acid or a salt thereof.

**9.** A process according to claim 1 for the preparation of 2-(1-benzylimidazol-2-yl)-1-hydroxyethane-1,1-diphosphonic acid or a salt thereof.

**10.** A process according to claim 1 for the preparation of 2-(1-methylimidazol-4-yl)-1-hydroxyethane-1,1-diphosphonic acid or a salt thereof.

**11.** A process according to claim 1 for the preparation of 1-amino-2-(1-methylimidazol-4-yl)ethane-1,1-diphosphonic acid or a salt thereof.

**12.** A process according to claim 1 for the preparation of 1-amino-2-(1-benzylimidazol-4-yl)ethane-1,1-diphosphonic acid or a salt thereof.

**13.** A process according to claim 1 for the preparation of 2-(1-methylimidazol-2-yl)ethane-1,1-diphosphonic acid or a salt thereof.

**14.** A process according to claim 1 for the preparation of 2-(1-benzylimidazol-2-yl)ethane-1,1-diphosphonic acid or a salt thereof.

**15.** A process according to claim 1 for the preparation of 2-(imidazol-1-yl)-1-hydroxyethane-1,1-diphosphonic acid or a salt thereof.

**16.** A process according to claim 1 for the preparation of 2-(imidazol-1-yl)ethane-1,1-diphosphonic acid or a salt thereof.

**17.** A process according to claim 1 for the preparation of 2-(thiazol-2-yl)ethane-1,1-diphosphonic acid or a salt thereof.

**18.** A process according to claim 1 for the preparation of 2-(4H-1,2,4-triazol-4-yl)-1-hydroxyethane-1,1-diphosphonic acid or a salt thereof.

**19.** A process according to claim 1 for the preparation of 2-(imidazol-2-yl)ethane-1,1-diphosphonic acid or a salt thereof.

**20.** A process according to claim 1 for the preparation of 2-(1-methyl-1H-1,2,4-triazol-5-yl)ethane-1,1-diphosphonic acid or a salt thereof.

**21.** A process according to claim 1 for the preparation of 2-[2-methylimidazol-4(5)-yl]ethane-1,1-diphosphonic acid or a salt thereof.

**22.** A process according to claim 1 for the preparation of 2-[2-phenylimidazol-4(5)-yl]ethane-1,1-diphosphonic acid or a salt thereof.

**23.** A process according to claim 1 for the preparation of 2-(4,5-dimethylimidazol-1-yl)-1-hydroxyethane-1,1-diphosphonic acid or a salt thereof.

**24.** A process according to claim 1 for the preparation of 2-(2-methylimidazol-1-yl)-1-hydroxyethane-1,1-diphosphonic acid or a salt thereof.

**25.** A process for the preparation of a pharmaceutical composition, which comprises mixing a compound obtainable according to any one of claims 1 to 24 with conventional pharmaceutical excipients.

**Revendications**
**Revendications pour les Etats contractants suivants : AT, BE, CH, DE, FR, GB, IT, LI, LU, NL, SE**

**1.** Acides alcane-diphosphoniques substitués, en particulier acides hétéroarylalcane-diphosphoniques de formule

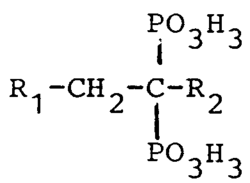

dans laquelle $R_1$ représente un groupe imidazolyle, 2H-1,2,3-, 1H-1,2,4- ou 4H-1,2,4-triazolyle, tétrazolyle , oxazolyle, isoxazolyle, oxadiazolyle, thiazolyle ou thiadiazolyle, qui est non substitué ou mono- ou di-substitué sur des carbones par des groupes alkyle inférieurs, par des groupes alcoxy inférieurs, par des groupes phényle qui peuvent eux-mêmes être mono- ou di-substitués par des groupes alkyle inférieurs, alcoxy inférieurs et/ou des halogènes, par des groupes hydroxy, par des groupes di(alkyle inférieur)-amino, par des groupes alkylthio inférieurs et/ou par des halogènes, et substitué sur un atome d'azote substituable par un groupe alkyle inférieur ou phényl-alkyle inférieur qui peut lui-même être mono- ou di-substitué dans la partie phényle par des groupes alkyle inférieurs, alcoxy inférieurs et/ou des halogènes, alk représente un groupe alkylène inférieur et $R_2$ l'hydrogène, un groupe hydroxy, amino, alkylthio inférieur ou un halogène, les groupes qualifiés d'"inférieurs" contenant jusqu'à 7 atomes de carbone inclus, avec les restrictions suivantes : $R_1$ ne peut représenter un groupe imidazole-4-yle, 2-méthylthiazole-4-yle, 2-méthylthiazole-5-yle et 1,2,5-thiadiazole-4-yle lorsque $R_2$ représente l'hydrogène, $R_1$ ne peut représenter un groupe imidazole-2-yle, 5(4)-méthyl-imidazole-4(5)-yle, 2H-1,2,3-triazole-4-yle, 1H-1,2,4-triazole-1-yle, 1H-1,2,4-triazole-3-yle, 5-éthoxy-, 5-éthoxy-2-méthyl-, 2-chloro-, 5-méthoxy- et 2,5-diméthyl-oxazole-4-yle, 3-méthyl- et 3-phénylisoxazole-5-yle, 3-méthyl-1,2,5-oxadiazole-4-yle, 2-méthyl-1,3,4-oxadiazole-5-yle, 3-phényl-1,2,4-oxadiazole-5-yle, 2-chloro- et 2-méthylthiazole-5-yle, 1,2,3- et 1,2,5-thiadiazole-4-yle, 1,2,3-thiadiazole-5-yle, 4-méthyl-1,2,3-thiadiazole-5-yle et 3-méthyl- et 3-phényl-1,2,4-thiadiazole-5-yle lorsque $R_2$ représente un groupe hydroxy, et $R_1$ ne peut représenter un groupe imidazole-4-yle lorsque $R_2$ représente un groupe amino ou diméthylamino, et leurs sels.

**2.** Composés de formule I dans laquelle $R_1$ représente un groupe imidazolyle, 2H-1,2,3- ou 4H-1,2,4-triazolyle, tétrazolyle, oxazolyle, isoxazolyle,oxadiazolyle, thiazolyle ou thiadiazolyle non substitué ou mono- ou di-substitué sur des carbones par des groupes alkyle inférieurs, alcoxy inférieurs, phényle lui-même non substitué ou mono- ou di-substitué par des groupes alkyle inférieurs, alcoxy inférieurs et/ou des halogènes, des groupes hydroxy, di-(alkyle inférieur)-amino, alkylthio inférieur et/ou des halogènes, et/ou substitué sur un atome d'azote substituable par un groupe alkyle inférieur ou phényl-alkyle

inférieur lui-même non substitué ou mono- ou di-substitué par des groupes alkyle inférieurs, alcoxy inférieurs et/ou des halogènes, et $R_2$ représente l'hydrogène, un groupe hydroxy, amino, alkylthio inférieur ou un halogène, et leurs sels.

**3.** Composés de formule I dans laquelle $R_1$ représente un groupe imidazolyle, 4H-1,2,4-triazole-4-yle ou thiazolyle non substitué ou mono- ou di-substitué sur des carbones par des groupes alkyle en C 1-C 4, alcoxy en C 1-C 4, phényle, hydroxy, di-(alkyle en C 1-C 4)-amino, alkylthio en C 1-C 4 et/ou des halogènes de numéro atomique allant jusqu'à 35 inclus, et/ou substitué sur l'azote par des groupes alkyle en C 1-C 4 ou phényl-alkyle en C 1-C 4, et $R_2$ représente un groupe hydroxy, l'hydrogène ou un groupe amino, et leurs sels.

**4.** Composés de formule I dans laquelle $R_1$ représente un groupe imidazole-2- ou -4-yle non substitué ou substitué sur le carbone par un groupe phényle ou sur l'azote ou le carbone par un groupe alkyle en C 1-C 4, un groupe thiazolyle non substitué ou un groupe 1H-1,2,4-triazolyle non substitué ou substitué à l'azote par un groupe alkyle en C 1-C 4, et $R_2$ représente un groupe hydroxy ou l'hydrogène, et leurs sels.

**5.** Composés selon la revendication 1, dans lesquels $R_1$ représente un groupe imidazole-1-yle, 2-, 4- ou 5-(alkyle en C 1-C 4)-imidazole-1-yle, 3-(alkyle en C 1-C 4)-1H-1,2,4-triazole-1-yle, 4H-triazole-4-yle, 3-(alkyle en C 1-C 4)-4H-1,2,4-triazole-4-yle ou 1H-tétrazole-1-yle, et alk et $R_2$ ont les significations indiquées dans la revendication 1, et leurs sels.

**6.** Composés de formule I dans laquelle $R_1$ représente un groupe imidazole-1-yle, 4H-1,2,4-triazole-4-yle ou tétrazole-1-yle non substitué ou substitué sur le carbone par un groupe phényle ou alkyle en C 1-C 4, et $R_2$ représente un groupe hydroxy ou l'hydrogène, et leurs sels.

**7.** L'acide 2-(1-méthylimidazole-2-yl)-1-hydroxyéthane-1,1-diphosphonique ou l'un de ses sels.

**8.** L'acide 2-(1-benzylimidazole-2-yl)-1-hydroxyéthane-1,1-diphosphonique ou l'un de ses sels.

**9.** L'acide 2-(1-méthylimidazole-4-yl)-1-hydroxyéthane-1,1-diphosphonique ou l'un de ses sels.

**10.** L'acide 1-amino-2-(1-méthylimidazole-4-yl)-éthane-1,1-diphosphonique ou l'un de ses sels.

**11.** L'acide 1-amino-2-(1-benzylimidazole-4-yl)-éthane-1,1-diphosphonique ou l'un de ses sels.

**12.** L'acide 2-(1-méthylimidazole-2-yl)-éthane-1,1-diphosphonique ou l'un de ses sels.

**13.** L'acide 2-(1-benzylimidazole-2-yl)-éthane-1,1-diphosphonique ou l'un de ses sels.

**14.** L'acide 2-(imidazole-1-yl)-1-hydroxyéthane-1,1-diphosphonique ou l'un de ses sels.

**15.** L'acide 2-(imidazole-1-yl)-éthane-1,1-diphosphonique ou l'un de ses sels.

**16.** L'acide 2-(4H-1,2,4-triazole-4-yl)-1-hydroxyéthane-1,1-diphosphonique ou l'un de ses sels.

**17.** L'acide 2-(thiazole-2-yl)-éthane-1,1-diphosphonique ou l'un de ses sels.

**18.** L'acide 2-(imidazole-2-yl)-éthane-1,1-diphosphonique ou l'un de ses sels.

**19.** L'acide 2-[2-méthylimidazole-4(5)-y1]-éthane-1,1-diphosphonique ou l'un de ses sels.

**20.** L'acide 2-[2-phénylimidazole-4(5)-yl]-éthane-1,1-diphosphonique ou l'un de ses sels.

**21.** L'acide 2-(4,5-diméthylimidazole-1-yl)-1-hydroxyéthane-1,1-diphosphonique ou l'un de ses sels.

**22.** L'acide 2-(2-méthylimidazole-1-yl)-1-hydroxyéthane-1,1-diphosphonique ou l'un de ses sels.

**23.** Un composé selon l'une des revendications 1, 4, 5 et 17 à 22 pour l'utilisation dans un procédé pour le traitement thérapeutique de l'organisme humain ou animal, par exemple en tant qu'agent régulateur du métabolisme du calcium et/ou agent anti-arthritique.

**24.** Un composé selon l'une des revendications 2, 3 et 6 à 16 pour l'utilisation dans un procédé pour le traitement thérapeutique de l'organisme humain ou animal, par exemple en tant qu'agent régulateur du métabolisme du calcium et/ou en tant qu'agent anti-arthritique.

**25.** Compositions pharmaceutiques contenant un composé selon l'une des revendications 1, 4, 5 et 17 à 23 avec des produits auxiliaires pharmaceutiques usuels.

**26.** Compositions pharmaceutiques contenant un composé selon l'une des revendications 2, 3, 6 à 16 et 24.

**27.** Procédé de préparation des composés de la revendication 1 et de leurs sels, caractérisé en ce que
a) dans un composé éventuellement protégé transitoirement sur un atome d'azote substituable du groupe $R_1$, et répondant à la formule

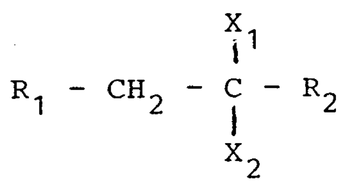

(II),

dans laquelle $X_1$ représente un groupe phosphono ayant subi une modification fonctionnelle et $X_2$ un groupe phosphono libre ou ayant subi une modification fonctionnelle, on convertit $X_1$ et le cas échéant $X_2$ en le groupe phosphono libre, ou bien
b) on fait réagir un composé éventuellement protégé transitoirement sur un atome d'azote substituable du groupe $R_1$, et répondant à la formule

$R_1$ - $CH_2$ - $X_3$ (III),

dans laquelle $X_3$ représente un groupe carboxy, carbamyle, iminoéther, iminoester ou cyano, avec l'acide phosphoreux et le trichlorure de phosphore : partant de composés de formule IV dans laquelle $X_3$ représente un groupe carbamyle, iminoéther, iminoester, cyano, on obtient, au traitement par hydrolyse, des composés de formule I dans laquelle $R_2$ représente un groupe amino, et si on le désire, on convertit un composé ainsi obtenu en un autre composé de formule I et/ou un composé obtenu à l'état libre en un sel ou un sel ainsi obtenu en le composé libre ou en un autre sel.

**28.** Procédé selon la revendication 27 caractérisé en ce que
a) dans un composé de formule

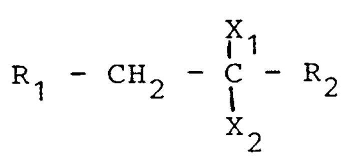

(II),

dans laquelle $X_1$ représente un groupe phosphono ayant subi une modification fonctionnelle et $X_2$ un groupe phosphono libre ou ayant subi une modification fonctionnelle, on convertit $X_1$ et le cas échéant $X_2$ en le groupe phosphono libre, ou bien
b) on fait réagir un composé de formule

$R_1$ - $CH_2$ - $X_3$ (III),

dans laquelle $X_3$ représente un groupe carboxy ou cyano, avec l'acide phosphoreux et le trichlorure de phosphore : partant de composés de formule III dans laquelle $X_3$ représente un groupe cyano, le traitement par hydrolyse donne des composés de formule I dans laquelle $R_2$ représente un groupe amino, et si on le désire, on convertit un composé ainsi obtenu en un autre composé de formule I et/ou un composé obtenu à l'état libre en un sel ou un sel ainsi obtenu en le composé libre ou en un autre sel.

**29.** Utilisation d'un composé selon l'une des revendications 1 à 22 pour la préparation d'un médicament approprié au traitement des maladies du métabolisme du calcium.

**Revendications pour les Etats contractants suivants : ES, GR**

**1.** Procédé de préparation d'acides alcane-diphosphoniques, en particulier d'acides hétéroarylalcane-diphosphoniques de formule

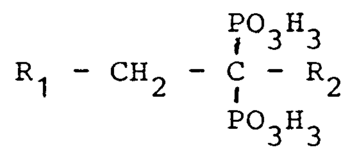

dans laquelle $R_1$ représente un groupe imidazolyle, 2H-1,2,3-, 1H-1,2,4- ou 4H-1,2,4-triazolyle, tétrazolyle, oxazolyle, isoxazolyle, oxadiazolyle, thiazolyle ou thiadiazolyle, qui peut être non substitué ou mono- ou di-substitué sur des carbones par des groupes alkyle inférieurs, par des groupes alcoxy inférieurs, par des groupes phényle eux-mêmes éventuellement mono- ou di-substitués par des groupes alkyle inférieurs, alcoxy inférieurs et/ou des halogènes, par des groupes hydroxy, par des groupes di-(alkyle inférieur)-amino, par des groupes alkylthio inférieurs et/ou par des halogènes, et substitué sur un atome d'azote substituable par un groupe alkyle inférieur ou par un groupe phényl-alkyle inférieur lui-même éventuellement mono- ou di-substitué dans la partie phényle par des groupes alkyle inférieurs, alcoxy inférieurs et/ou des halogènes, et $R_2$ représente l'hydrogène, un groupe hydroxy, amino, alkylthio inférieur ou un halogène, avec les restrictions suivantes : $R_1$ ne peut représenter un groupe imidazole-4-yle, 2-méthylthiazolyle-4-yle, 2-méthylthiazole-5-yle et 1,2,5-thiadiazole-4-yle lorsque $R_2$ représente l'hydrogène, $R_1$ ne peut représenter un groupe imidazole-2-yle, 5(4)-méthylimidazole-4(5)-yle, 2H-1,2,3-triazole-4-yle, 1H-1,2,4-triazole-1-yle, 1H-1,2,4-triazole-3-yle, 5-éthoxy-, 5-éthoxy-2-méthyl-, 2-chloro-, 5-méthoxy- et 2,5-diméthyloxazole-4-yle, 3-méthyl- et 3-phényl-isoxazole-5-yle, 3-méthyl-1,2,5-oxadiazole-4-yle, 2-méthyl-1,3,4-oxadiazole-5-yle, 3-phényl-1,2,4-oxadiazole-5-yle, 2-chloro- et 2-méthyl-thiazole-5-yle, 1,2,3- et 1,2,5-thiadiazole-4-yle, 1,2,3-thiadiazole-5-yle, 4-méthyl-1,2,3-thiadiazole-5-yle et 3-méthyl- et 3-phényl-1,2,4-thiadiazole-5-yle lorsque $R_2$ représente un groupe hydroxy, et $R_1$ ne peut représenter un groupe imidazole-4-yle lorsque $R_2$ représente un groupe amino ou diméthylamino, et de leurs sels, caractérisé en ce que

a) dans un composé éventuellement protégé transitoirement sur un atome d'azote substituable du groupe $R_1$, et répondant à la formule

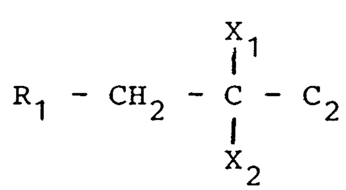

(II),

dans laquelle $X_1$ représente un groupe phosphono ayant subi une modification fonctionnelle et $X_2$ un groupe phosphono libre ou ayant subi une modification fonctionnelle, on convertit $X_1$ et le cas échéant $X_2$ en groupe phosphono libre, ou bien

b) on fait réagir un composé éventuellement protégé transitoirement sur un atome d'azote substituable du groupe $R_1$, et répondant à la formule

$R_1 - CH_2 - X_3$ (III),

dans laquelle $X_3$ représente un groupe carboxy, carbamyle, iminoéther, iminoester ou cyano, avec l'acide phosphoreux et le trichlorure de phosphore : partant de composés de formule IV dans laquelle $X_3$ représente un groupe carbamyle, iminoéther, iminoester, cyano, on obtient au traitement par hydrolyse des composés de formule I dans laquelle $R_2$ représente un groupe amino, et si on le désire, on convertit un composé ainsi obtenu en un autre composé de formule I et/ou un composé obtenu à l'état libre en un sel ou un sel obtenu en le composé libre ou en un autre sel.

**2.** Procédé selon la revendication 1, pour la préparation de composés de formule I dans laquelle $R_1$ représente un groupe imidazolyle, 2H-1 ,2,3- ou 4H-1,2,4-triazolyle, tétrazolyle, oxazolyle, isoxazolyle, oxadiazolyle, thiazolyle ou thiadiazolyle non substitué ou mono- ou di-substitué sur des carbones par des groupes alkyle inférieurs, alcoxy inférieurs, phényle eux-mêmes non substitués ou mono- ou di-substitués par des groupes alkyle inférieurs, alcoxy inférieurs et/ou des halogènes, des groupes hydroxy, di-(alkyle inférieur)-amino, alkylthio inférieurs et/ou des halogènes, et/ou substitué sur un atome d'azote substituable par un groupe alkyle inférieur ou un groupe phényl-alkyle inférieur lui-même non substitué ou mono- ou di-substitué par des groupes alkyle inférieurs, alcoxy inférieurs et/ou des halogènes, et $R_2$ représente l'hydrogène, un groupe hydroxy, amino, alkylthio inférieur ou un halogène, et de leurs sels.

**3.** Procédé selon la revendication 1 pour la préparation de composés de formule I dans laquelle $R_1$ représente un groupe imidazolyle, 4H-1,2,4-triazolyle ou thiazolyle non substitué ou mono- ou di-substitué sur des carbones par des groupes alkyle en C 1-C 4, alcoxy en C 1-C 4, phényle, hydroxy, di-(alkyle en C 1-C 4)-amino, alkylthio en C 1-C 4 et/ou- des halogènes de numéro atomique allant jusqu'à 35 inclus et/ou substitué à l'azote par un groupe alkyle en C 1-C 4 ou phényl-alkyle en C 1-C 4, et $R_2$ représente un groupe hydroxy, l'hydrogène ou un groupe amino, et de leurs sels.

**4.** Procédé selon la revendication 1 pour la préparation de composés de formule I dans laquelle $R_1$ représente un groupe imidazole-2- ou -4-yle non substitué ou substitué sur le carbone par un groupe phényle ou substitué sur le carbone ou l'azote par un groupe alkyle en C 1-C 4, un groupe thiazolyle non substitué ou un groupe 1H-1,2,4-triazolyle non substitué ou substitué à l'azote par un groupe alkyle en C 1-C 4, et $R_2$ représente un groupe hydroxy ou, en second lieu, l'hydrogène, et de leurs sels.

**5.** Procédé selon la revendication 1, pour la préparation de composés de formule I dans laquelle $R_1$ représente un groupe imidazole-1-yle, 1H-1,2,4-triazole-1-yle, 4H-1,2,4-triazole-4-yle, ou tétrazole-1-yle non substitué ou substitué sur des carbones par des groupes phényle ou alkyle en C 1-C 4, et $R_2$ représente un groupe hydroxy ou, en second lieu, l'hydrogène, et de leurs sels.

**6.** Procédé selon la revendication 1, pour la préparation de composés de formule I dans laquelle $R_1$ représente un groupe imidazole-1-yle, 2-, 4- ou 5-(alkyle en C 1-C 4)-imidazole-1-yle, 3-(alkyle en C 1-C 4)-1H-1,2,4-triazole-1-yle, 4H-triazole-4-yle, 3-(alkyle en C 1-C 4)-4H-1,2,4-triazole-4-yle ou 1H-tétrazole-1-yle, et alk et $R_2$ ont les significations indiquées dans la revendication 1, et de leurs sels.

**7.** Procédé selon la revendication 1 pour la préparation des composés de formule I dans laquelle $R_1$ représente un groupe imidazole non substitué ou substitué par des groupes alkyle en C 1-C 4 et $R_2$ représente un groupe hydroxy ou l'hydrogène et de leurs sels.

**8.** Procédé selon la revendication 1, pour la préparation de l'acide 2-(1-méthylimidazole-2-yl)-1-hydroxyéthane-1,1-diphosphonique ou de l'un de ses sels.

**9.** Procédé selon la revendication 1, pour la préparation de l'acide 2-(1-benzylimidazole-2-yl)-1-hydroxyéthane-1,1-diphosphonique ou de l'un de ses sels.

**10.** Procédé selon la revendication 1, pour la préparation de l'acide 2-(1-méthylimidazole-4-yl)-1-hydroxyéthane-1,1-diphosphonique ou de l'un de ses sels.

**11.** Procédé selon la revendication 1, pour la préparation de l'acide 1-amino-2-(1-méthylimidazole-4-yl)-éthane-1,1-diphosphonique ou de l'un de ses sels.

12. Procédé selon la revendication 1, pour la préparation de l'acide 1-amino-2-(1-benzylimidazole-4-yl)-éthane-1,1-diphosphonique ou de l'un de ses sels.

13. Procédé selon la revendication 1, pour la préparation de l'acide 2-(1-méthylimidazole-2-yl)-éthane-1,1-diphosphonique ou de l'un de ses sels.

14. Procédé selon la revendication 1, pour la préparation de l'acide 2-(1-benzylimidazole-2-yl)-éthane-1,1-diphosphonique ou de l'un de ses sels.

15. Procédé selon la revendication 1, pour la préparation de l'acide 2-(imidazole-1-yl)-1-hydroxyéthane-1,1-diphosphonique ou de l'un de ses sels.

16. Procédé selon la revendication 1, pour la préparation de l'acide 2-(imidazole-1-yl)-éthane-1,1-diphosphonique ou de l'un de ses sels.

17. Procédé selon la revendication 1, pour la préparation de l'acide 2-(thiazole-2-yl)-éthane-1,1-diphosphonique ou de l'un de ses sels.

18. Procédé selon la revendication 1, pour la préparation de l'acide 2-(4H-1,2,4-triazole-4-yl)-1-hydroxyéthane-1,1-diphosphonique ou de l'un de ses sels.

19. Procédé selon la revendication 1, pour la préparation de l'acide 2-(imidazole-2-yl)-éthane-1,1-diphosphonique ou de l'un de ses sels.

20. Procédé selon la revendication 1, pour la préparation de l'acide 2-(1-méthyl-1H-1,2,4-triazole-5-yl)-éthane-1,1-diphosphonique ou de l'un de ses sels.

21. Procédé selon la revendication 1, pour la préparation de l'acide 2-[2-méthylimidazole-4(5)-yl]-éthane-1,1-diphosphonique ou de l'un de ses sels.

22. Procédé selon la revendication 1, pour la préparation de l'acide 2-[2-phénylimidazole-4(5)-yl]-éthane-1,1-diphosphonique ou de l'un de ses sels.

23. Procédé selon la revendication 1, pour la préparation de l'acide 2-(4,5-diméthylimidazole-1-yl)-1-hydroxyéthane-1,1-diphosphonique ou de l'un de ses sels.

24. Procédé selon la revendication 1, pour la préparation de l'acide 2-(2-méthylimidazole-1-yl)-1-hydroxyéthane-1,1-diphosphonique ou de l'un de ses sels.

25. Procédé de préparation de compositions pharmaceutiques, caractérisé en ce que l'on mélange un composé obtenu selon l'une des revendications 1 à 24 avec des produits auxiliaires pharmaceutiques usuels.